(19) **Europäisches Patentamt / European Patent Office / Office européen des brevets**

(11) **EP 4 692 178 A1**

(12) **EUROPEAN PATENT APPLICATION**
published in accordance with Art. 153(4) EPC

(43) Date of publication:
**11.02.2026 Bulletin 2026/07**

(21) Application number: **24780794.4**

(22) Date of filing: **29.03.2024**

(51) International Patent Classification (IPC):
$C08J\ 3/12$ (2006.01)  $A23L\ 29/262$ (2016.01)
$A61K\ 9/20$ (2006.01)  $A61K\ 31/375$ (2006.01)
$A61K\ 47/38$ (2006.01)

(52) Cooperative Patent Classification (CPC):
A23L 29/262; A61K 9/20; A61K 31/375;
A61K 47/38; C08J 3/12

(86) International application number:
**PCT/JP2024/013018**

(87) International publication number:
**WO 2024/204699 (03.10.2024 Gazette 2024/40)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **31.03.2023 JP 2023058328**

(71) Applicant: **Asahi Kasei Kabushiki Kaisha
Tokyo 1000006 (JP)**

(72) Inventors:
• **TAMARI, Kaede
Tokyo 100-0006 (JP)**

• **TSURU, Kyosuke
Tokyo 100-0006 (JP)**
• **YOSHIDA, Ayumi
Tokyo 100-0006 (JP)**
• **MAEDA, Kazuki
Tokyo 100-0006 (JP)**
• **HONDA, Yosuke
Tokyo 100-0006 (JP)**

(74) Representative: **Hoffmann Eitle
Patent- und Rechtsanwälte PartmbB
Arabellastraße 30
81925 München (DE)**

(54) **CELLULOSE POWDER AND MOLDED BODY**

(57) The present invention provides cellulose powder which is capable of obtaining a smaller molded article having excellent hardness and a low abrasion degree without changing the amount of an active component such as a drug while the variation in mass is reduced and the disintegrability is satisfactorily maintained, and a molded article formed of the cellulose powder. The present invention provides cellulose powder in which a ratio Y ($Cv/D_{50}$) of a water absorption rate Cv to an average particle diameter $D_{50}$ is 0.12 $g^2/(s \cdot \mu m)$ or greater, and a loose bulk density X is 0.135 $g/cm^3$ or less, cellulose powder in which a relationship between a ratio Y ($Cv/D_{50}$) of a water absorption rate Cv to an average particle diameter $D_{50}$ and a loose bulk density X satisfies Expression (1-1) [$Y \geq 1.5 \times X - 0.06$], and a molded article including one or more active components, and any of the cellulose powders described above.

EP 4 692 178 A1

**Description**

TECHNICAL FIELD

The present invention relates to cellulose powder and a molded article.
Priority is claimed on Japanese Patent Application No. 2023-058328, filed March 31, 2023, the content of which is incorporated herein by reference.

BACKGROUND ART

In the related art, in the fields of pharmaceuticals, foods, and other chemical industries, preparation of a molded article containing an active component has been widely carried out by using cellulose powder such as crystalline cellulose or powdered cellulose as an excipient. Such cellulose powder is required to have particularly satisfactory moldability and to be easily taken in by reducing the size of the molded article without changing the amount of active components such as drugs.
For example, Patent Document 1 discloses an absorbent article having an excellent permeation rate, and water-absorbent resin particles and an absorber that provide the absorbent article. Specifically, the absorbent article includes an absorber, the absorber contains water-absorbent resin particles, the water-absorbent resin particles have a packed bulk density of 0.500 $g/cm^3$ or greater, and a change in amount of the packed bulk density is 0.020 $g/cm^3$ or greater in a case of moisture absorption at a temperature of 30°C and a relative humidity of 80% for 1 hour.
Patent Document 2 discloses cellulose powder which has excellent compression moldability, uniformly maintains a component having pressure-sensitive adhesiveness during granulation, can make a particle size distribution of granules sharp, reduces the disintegration time of a molded article, and can impart temporally stabilized disintegrability. Specifically, Patent Document 2 discloses that in the cellulose powder, the sharpness of the particle size distribution is 1.5 to 2.9, and the total amount of organic carbon derived from residual impurities is greater than 0.07% and 0.25% or less.

Citation List

Patent Documents

Patent Document 1: Japanese Unexamined Patent Application, First Publication No. 2020-121298
Patent Document 2: Japanese Patent No. 6273052

SUMMARY OF INVENTION

Technical Problem

However, Patent Document 1 discloses resin particles having a structural unit derived from at least one selected from the group consisting of (meth)acrylic acid and a salt thereof, as a material that exhibits an excellent permeation rate with respect to a liquid containing water as a main component and can maintain the liquid, and has not examined cellulose powder.
In addition, Patent Document 2 discloses only a molded article obtained by a wet tableting method, and has not examined a direct tableting method. In addition, the reduction in size of the molded article has also not examined.
The present invention has been made in consideration of the above-described circumstances, and provides cellulose powder which is capable of obtaining a smaller molded article having excellent hardness and a low abrasion degree without changing the amount of an active component such as a drug while the variation in mass is reduced and the disintegrability is satisfactorily maintained, and a molded article formed of the cellulose powder.

Solution to Problem

That is, the present invention includes the following aspects.

[1] Cellulose powder, in which a ratio Y ($Cv/D_{50}$) of a water absorption rate Cv to an average particle diameter $D_{50}$ is 0.12 $g^2/(s \cdot \mu m)$ or greater, and a loose bulk density X is 0.135 $g/cm^3$ or less.
[2] Cellulose powder, in which a relationship between a ratio Y ($Cv/D_{50}$) of a water absorption rate Cv to an average particle diameter $D_{50}$ and a loose bulk density X satisfies Expression (1-1).

$$Y \geq 1.5 \times X - 0.06 \cdots\cdots \text{Expression (1-1)}$$

[3] The cellulose powder according to [2], in which the ratio Y ($Cv/D_{50}$) of the water absorption rate Cv to the average particle diameter $D_{50}$ is 0.12 $g^2/(s \cdot \mu m)$ or greater, and the loose bulk density X is 0.135 $g/cm^3$ or less.

[4] The cellulose powder according to any one of [1] to [3], in which a ratio $F/D_{50}$ of a cohesive force F to the average particle diameter $D_{50}$ is 0.01N%/$\mu m$ or greater.

[5] The cellulose powder according to any one of [1] to [4], in which a powder dynamic friction angle is 30° or greater.

[6] The cellulose powder according to any one of [1] to [5], in which a primary particle ratio is 20% or greater.

[7] The cellulose powder according to any one of [1] to [6], in which a ratio L/D of primary particles is 2.0 or greater.

[8] The cellulose powder according to any one of [1] to [7], in which a compression ratio is 40% or greater.

[9] The cellulose powder according to any one of [1] to [8], in which an average degree of polymerization is 100 or greater and 450 or less.

[10] The cellulose powder according to any one of [1] to [9], in which the average particle diameter $D_{50}$ is 15 $\mu m$ or greater and 300 $\mu m$ or less.

[11] The cellulose powder according to any one of [1] to [10], in which the water absorption rate Cv is 2.0 $g^2/s$ or greater and 12.0 $g^2/s$ or less.

[12] The cellulose powder according to any one of [1] to [11], in which a cohesive force F is 30 N% or greater and 100 N% or less.

[13] A molded article including: one or more active components; and the cellulose powder according to any one of [1] to [12].

[14] The molded article according to [13], in which the active component is a pharmaceutically active component.

[15] The molded article according to [13], in which the active component is an active component for food.

[16] The molded article according to any one of [13] to [15], in which the molded article is a tablet.

Advantageous Effects of Invention

According to the cellulose powder of the above-described aspect, it is possible to provide cellulose powder which is capable of obtaining a smaller molded article having excellent hardness and a low abrasion degree without changing the amount of an active component such as a drug while the variation in mass is reduced and the disintegrability is satisfactorily maintained.

DESCRIPTION OF EMBODIMENTS

<Cellulose powder>

Cellulose powder according to one embodiment of the present invention (hereinafter, referred to as "the present embodiment") satisfies "a ratio Y ($Cv/D_{50}$) of a water absorption rate Cv to an average particle diameter $D_{50}$ is 0.12 $g^2/(s \cdot \mu m)$ or greater and a loose bulk density X is 0.135 $g/cm^3$ or less" or "a relationship between a ratio Y ($Cv/D_{50}$) of a water absorption rate Cv to an average particle diameter $D_{50}$ and a loose bulk density X is $Y \geq 1.5 \times X - 0.06$".

Since the cellulose powder of the present embodiment has the above-described configuration, it is possible to obtain a smaller molded article having excellent hardness and a low abrasion degree without changing the amount of an active component such as a drug while the variation in mass is reduced and the disintegrability is satisfactorily maintained.

The cellulose powder in the specification of the present application is generally referred to as crystalline cellulose, powdered cellulose, or the like, and is suitably used as a pharmaceutical additive or a food additive. Among these, crystalline cellulose is preferable as the cellulose powder.

Known examples of the crystalline cellulose include microcrystalline cellulose described in the 9th edition of the Food Additive Standards, crystalline cellulose described in Japanese Pharmacopoeia (18th edition), and crystalline cellulose described in the United States Pharmacopeia, European Pharmacopoeia, and the like.

[Average degree of polymerization]

The average degree of polymerization of the cellulose powder of the present embodiment is 100 or greater and 450 or less, preferably 150 or greater and 450 or less, more preferably 200 or greater and 450 or less, still more preferably 200 or greater and 350 or less, even still more preferably 200 or greater and 310 or less, and particularly preferably 200 or greater and 300 or less. In a case where the average degree of polymerization of the cellulose powder is in the above-described ranges, a molded article having satisfactory moldability is obtained.

The average degree of polymerization of the cellulose powder is measured by the method described below.

About 1.3 g of cellulose powder (about 0.25 g in a case of powdered cellulose) is precisely weighed as a sample and placed in a 125 mL Erlenmeyer flask, and 25 mL of water and 25 mL of a 1 mol/L copper ethylenediamine test solution are each accurately added thereto. The flask is tightly sealed immediately after passing through nitrogen, and the mixture is dissolved while being mixed with a shaker. The appropriate amount of the liquid is accurately weighed, and a test is performed at $25 \pm 0.1°C$ using a capillary viscometer in which an approximate constant (K) of the viscometer is 0.03 to determine a kinematic viscosity v by the first method <2.53> of the viscosity measuring method. Separately, 25 mL of water and 25 mL of a 1 mol/L copper ethylenediamine test solution are accurately weighed, and a test is performed on the mixed solution using a capillary viscometer in which an approximate constant (K) of the viscometer is 0.01 to determine a kinematic viscosity v0 by the same method. A relative viscosity $\eta_{rel}$ of the cellulose powder is determined by the following equation.

$$\eta_{rel} = v/v0$$

As listed in Tables 1 and 2, a product [η]C of a limiting viscosity [η] (mL/g) and a concentration C (g/100 mL) is obtained from the relative viscosity $\eta_{rel}$, and an average degree P of polymerization is calculated by the following equation. Further, in the following equation, MT represents a weighed amount (g) of the cellulose powder in terms of a dry substance.

$$P = 95 \ [\eta]C/MT$$

[Table 1]

**EP 4 692 178 A1**

Table for calculating the product $[\eta]C$ of limiting viscosity and concentration from relative viscosity $\eta_{rel}$

| $\eta_{rel}$ | $[\eta]\,C$ | | | | | | | | | |
|---|---|---|---|---|---|---|---|---|---|---|
| | 0.00 | 0.01 | 0.02 | 0.03 | 0.04 | 0.05 | 0.06 | 0.07 | 0.08 | 0.09 |
| 1.1 | 0.098 | 0.106 | 0.115 | 0.125 | 0.134 | 0.143 | 0.152 | 0.161 | 0.170 | 0.180 |
| 1.2 | 0.189 | 0.198 | 0.207 | 0.216 | 0.225 | 0.233 | 0.242 | 0.250 | 0.259 | 0.268 |
| 1.3 | 0.276 | 0.285 | 0.293 | 0.302 | 0.310 | 0.318 | 0.326 | 0.334 | 0.342 | 0.350 |
| 1.4 | 0.358 | 0.367 | 0.375 | 0.383 | 0.391 | 0.399 | 0.407 | 0.414 | 0.422 | 0.430 |
| 1.5 | 0.437 | 0.445 | 0.453 | 0.460 | 0.468 | 0.476 | 0.484 | 0.491 | 0.499 | 0.507 |
| 1.6 | 0.515 | 0.522 | 0.529 | 0.536 | 0.544 | 0.551 | 0.558 | 0.566 | 0.573 | 0.580 |
| 1.7 | 0.587 | 0.595 | 0.602 | 0.608 | 0.615 | 0.622 | 0.629 | 0.636 | 0.642 | 0.649 |
| 1.8 | 0.656 | 0.663 | 0.670 | 0.677 | 0.683 | 0.690 | 0.697 | 0.704 | 0.710 | 0.717 |
| 1.9 | 0.723 | 0.730 | 0.736 | 0.743 | 0.749 | 0.756 | 0.762 | 0.769 | 0.775 | 0.782 |
| | | | | | | | | | | |
| 2.0 | 0.788 | 0.795 | 0.802 | 0.809 | 0.815 | 0.821 | 0.827 | 0.833 | 0.840 | 0.846 |
| 2.1 | 0.852 | 0.858 | 0.864 | 0.870 | 0.876 | 0.882 | 0.888 | 0.894 | 0.900 | 0.906 |
| 2.2 | 0.912 | 0.918 | 0.924 | 0.929 | 0.935 | 0.941 | 0.948 | 0.953 | 0.959 | 0.965 |
| 2.3 | 0.971 | 0.976 | 0.983 | 0.988 | 0.994 | 1.000 | 1.006 | 1.011 | 1.017 | 1.022 |
| 2.4 | 1.028 | 1.033 | 1.039 | 1.044 | 1.050 | 1.056 | 1.061 | 1.067 | 1.072 | 1.078 |
| 2.5 | 1.083 | 1.089 | 1.094 | 1.100 | 1.105 | 1.111 | 1.116 | 1.121 | 1.126 | 1.131 |
| 2.6 | 1.137 | 1.142 | 1.147 | 1.153 | 1.158 | 1.163 | 1.169 | 1.174 | 1.179 | 1.184 |
| 2.7 | 1.190 | 1.195 | 1.200 | 1.205 | 1.210 | 1.215 | 1.220 | 1.225 | 1.230 | 1.235 |
| 2.8 | 1.240 | 1.245 | 1.250 | 1.255 | 1.260 | 1.265 | 1.270 | 1.275 | 1.280 | 1.285 |
| 2.9 | 1.290 | 1.295 | 1.300 | 1.305 | 1.310 | 1.314 | 1.319 | 1.324 | 1.329 | 1.333 |
| | | | | | | | | | | |
| 3.0 | 1.338 | 1.343 | 1.348 | 1.352 | 1.357 | 1.362 | 1.367 | 1.371 | 1.376 | 1.381 |
| 3.1 | 1.386 | 1.390 | 1.395 | 1.400 | 1.405 | 1.409 | 1.414 | 1.418 | 1.423 | 1.427 |
| 3.2 | 1.432 | 1.436 | 1.441 | 1.446 | 1.450 | 1.455 | 1.459 | 1.464 | 1.468 | 1.473 |
| 3.3 | 1.477 | 1.482 | 1.486 | 1.491 | 1.496 | 1.500 | 1.504 | 1.508 | 1.513 | 1.517 |
| 3.4 | 1.521 | 1.525 | 1.529 | 1.533 | 1.537 | 1.542 | 1.546 | 1.550 | 1.554 | 1.558 |
| 3.5 | 1.562 | 1.566 | 1.570 | 1.575 | 1.579 | 1.583 | 1.587 | 1.591 | 1.595 | 1.600 |
| 3.6 | 1.604 | 1.608 | 1.612 | 1.617 | 1.621 | 1.625 | 1.629 | 1.633 | 1.637 | 1.642 |
| 3.7 | 1.646 | 1.650 | 1.654 | 1.658 | 1.662 | 1.666 | 1.671 | 1.675 | 1.679 | 1.683 |
| 3.8 | 1.687 | 1.691 | 1.695 | 1.700 | 1.704 | 1.708 | 1.712 | 1.715 | 1.719 | 1.723 |
| 3.9 | 1.727 | 1.731 | 1.735 | 1.739 | 1.742 | 1.746 | 1.750 | 1.754 | 1.758 | 1.762 |
| | | | | | | | | | | |
| 4.0 | 1.765 | 1.769 | 1.773 | 1.777 | 1.781 | 1.785 | 1.789 | 1.792 | 1.796 | 1.800 |
| 4.1 | 1.804 | 1.808 | 1.811 | 1.815 | 1.819 | 1.822 | 1.826 | 1.830 | 1.833 | 1.837 |
| 4.2 | 1.841 | 1.845 | 1.848 | 1.852 | 1.856 | 1.859 | 1.863 | 1.867 | 1.870 | 1.874 |
| 4.3 | 1.878 | 1.882 | 1.885 | 1.889 | 1.893 | 1.896 | 1.900 | 1.904 | 1.907 | 1.911 |
| 4.4 | 1.914 | 1.918 | 1.921 | 1.925 | 1.929 | 1.932 | 1.936 | 1.939 | 1.943 | 1.946 |
| 4.5 | 1.950 | 1.954 | 1.957 | 1.961 | 1.964 | 1.968 | 1.971 | 1.975 | 1.979 | 1.982 |
| 4.6 | 1.986 | 1.989 | 1.993 | 1.996 | 2.000 | 2.003 | 2.007 | 2.010 | 2.013 | 2.017 |
| 4.7 | 2.020 | 2.023 | 2.027 | 2.030 | 2.033 | 2.037 | 2.040 | 2.043 | 2.047 | 2.050 |
| 4.8 | 2.053 | 2.057 | 2.060 | 2.063 | 2.067 | 2.070 | 2.073 | 2.077 | 2.080 | 2.083 |
| 4.9 | 2.087 | 2.090 | 2.093 | 2.097 | 2.100 | 2.103 | 2.107 | 2.110 | 2.113 | 2.116 |
| | | | | | | | | | | |
| 5.0 | 2.119 | 2.122 | 2.125 | 2.129 | 2.132 | 2.135 | 2.139 | 2.142 | 2.145 | 2.148 |
| 5.1 | 2.151 | 2.154 | 2.158 | 2.160 | 2.164 | 2.167 | 2.170 | 2.173 | 2.176 | 2.180 |
| 5.2 | 2.183 | 2.186 | 2.190 | 2.192 | 2.195 | 2.197 | 2.200 | 2.203 | 2.206 | 2.209 |
| 5.3 | 2.212 | 2.215 | 2.218 | 2.221 | 2.224 | 2.227 | 2.230 | 2.233 | 2.236 | 2.240 |
| 5.4 | 2.243 | 2.246 | 2.249 | 2.252 | 2.255 | 2.258 | 2.261 | 2.264 | 2.267 | 2.270 |
| 5.5 | 2.273 | 2.276 | 2.279 | 2.282 | 2.285 | 2.288 | 2.291 | 2.294 | 2.297 | 2.300 |
| 5.6 | 2.303 | 2.306 | 2.309 | 2.312 | 2.315 | 2.318 | 2.320 | 2.324 | 2.326 | 2.329 |
| 5.7 | 2.332 | 2.335 | 2.338 | 2.341 | 2.344 | 2.347 | 2.350 | 2.353 | 2.355 | 2.358 |
| 5.8 | 2.361 | 2.364 | 2.367 | 2.370 | 2.373 | 2.376 | 2.379 | 2.382 | 2.384 | 2.387 |
| 5.9 | 2.390 | 2.393 | 2.396 | 2.400 | 2.403 | 2.405 | 2.408 | 2.411 | 2.414 | 2.417 |

[Table 2]

## Table for calculating the product $[\eta]C$ of limiting viscosity and concentration from relative viscosity $\eta_{rel}$ (continued)

| $\eta_{rel}$ | 0.00 | 0.01 | 0.02 | 0.03 | 0.04 | 0.05 | 0.06 | 0.07 | 0.08 | 0.09 |
|---|---|---|---|---|---|---|---|---|---|---|
| 6.0 | 2.419 | 2.422 | 2.425 | 2.428 | 2.431 | 2.433 | 2.436 | 2.439 | 2.442 | 2.444 |
| 6.1 | 2.447 | 2.450 | 2.453 | 2.456 | 2.458 | 2.461 | 2.464 | 2.467 | 2.470 | 2.472 |
| 6.2 | 2.475 | 2.478 | 2.481 | 2.483 | 2.486 | 2.489 | 2.492 | 2.494 | 2.497 | 2.500 |
| 6.3 | 2.503 | 2.505 | 2.508 | 2.511 | 2.513 | 2.516 | 2.518 | 2.521 | 2.524 | 2.526 |
| 6.4 | 2.529 | 2.532 | 2.534 | 2.537 | 2.540 | 2.542 | 2.545 | 2.547 | 2.550 | 2.553 |
| 6.5 | 2.555 | 2.558 | 2.561 | 2.563 | 2.566 | 2.568 | 2.571 | 2.574 | 2.576 | 2.579 |
| 6.6 | 2.581 | 2.584 | 2.587 | 2.590 | 2.592 | 2.595 | 2.597 | 2.600 | 2.603 | 2.605 |
| 6.7 | 2.608 | 2.610 | 2.613 | 2.615 | 2.618 | 2.620 | 2.623 | 2.625 | 2.627 | 2.630 |
| 6.8 | 2.633 | 2.635 | 2.637 | 2.640 | 2.643 | 2.645 | 2.648 | 2.650 | 2.653 | 2.655 |
| 6.9 | 2.658 | 2.660 | 2.663 | 2.665 | 2.668 | 2.670 | 2.673 | 2.675 | 2.678 | 2.680 |
| 7.0 | 2.683 | 2.685 | 2.687 | 2.690 | 2.693 | 2.695 | 2.698 | 2.700 | 2.702 | 2.705 |
| 7.1 | 2.707 | 2.710 | 2.712 | 2.714 | 2.717 | 2.719 | 2.721 | 2.724 | 2.726 | 2.729 |
| 7.2 | 2.731 | 2.733 | 2.736 | 2.738 | 2.740 | 2.743 | 2.745 | 2.748 | 2.750 | 2.752 |
| 7.3 | 2.755 | 2.757 | 2.760 | 2.762 | 2.764 | 2.767 | 2.769 | 2.771 | 2.774 | 2.776 |
| 7.4 | 2.779 | 2.781 | 2.783 | 2.786 | 2.788 | 2.790 | 2.793 | 2.795 | 2.798 | 2.800 |
| 7.5 | 2.802 | 2.805 | 2.807 | 2.809 | 2.812 | 2.814 | 2.816 | 2.819 | 2.821 | 2.823 |
| 7.6 | 2.826 | 2.828 | 2.830 | 2.833 | 2.835 | 2.837 | 2.840 | 2.842 | 2.844 | 2.847 |
| 7.7 | 2.849 | 2.851 | 2.854 | 2.856 | 2.858 | 2.860 | 2.863 | 2.865 | 2.868 | 2.870 |
| 7.8 | 2.873 | 2.875 | 2.877 | 2.879 | 2.881 | 2.884 | 2.887 | 2.889 | 2.891 | 2.893 |
| 7.9 | 2.895 | 2.898 | 2.900 | 2.902 | 2.905 | 2.907 | 2.909 | 2.911 | 2.913 | 2.915 |
| 8.0 | 2.918 | 2.920 | 2.922 | 2.924 | 2.926 | 2.928 | 2.931 | 2.933 | 2.935 | 2.937 |
| 8.1 | 2.939 | 2.942 | 2.944 | 2.946 | 2.948 | 2.950 | 2.952 | 2.955 | 2.957 | 2.959 |
| 8.2 | 2.961 | 2.963 | 2.966 | 2.968 | 2.970 | 2.972 | 2.974 | 2.976 | 2.979 | 2.981 |
| 8.3 | 2.983 | 2.985 | 2.987 | 2.990 | 2.992 | 2.994 | 2.996 | 2.998 | 3.000 | 3.002 |
| 8.4 | 3.004 | 3.006 | 3.008 | 3.010 | 3.012 | 3.015 | 3.017 | 3.019 | 3.021 | 3.023 |
| 8.5 | 3.025 | 3.027 | 3.029 | 3.031 | 3.033 | 3.035 | 3.037 | 3.040 | 3.042 | 3.044 |
| 8.6 | 3.046 | 3.048 | 3.050 | 3.052 | 3.054 | 3.056 | 3.058 | 3.060 | 3.062 | 3.064 |
| 8.7 | 3.067 | 3.069 | 3.071 | 3.073 | 3.075 | 3.077 | 3.079 | 3.081 | 3.083 | 3.085 |
| 8.8 | 3.087 | 3.089 | 3.092 | 3.094 | 3.096 | 3.098 | 3.100 | 3.102 | 3.104 | 3.106 |
| 8.9 | 3.108 | 3.110 | 3.112 | 3.114 | 3.116 | 3.118 | 3.120 | 3.122 | 3.124 | 3.126 |
| 9.0 | 3.128 | 3.130 | 3.132 | 3.134 | 3.136 | 3.138 | 3.140 | 3.142 | 3.144 | 3.146 |
| 9.1 | 3.148 | 3.150 | 3.152 | 3.154 | 3.156 | 3.158 | 3.160 | 3.162 | 3.164 | 3.166 |
| 9.2 | 3.168 | 3.170 | 3.172 | 3.174 | 3.176 | 3.178 | 3.180 | 3.182 | 3.184 | 3.186 |
| 9.3 | 3.188 | 3.190 | 3.192 | 3.194 | 3.196 | 3.198 | 3.200 | 3.202 | 3.204 | 3.206 |
| 9.4 | 3.208 | 3.210 | 3.212 | 3.214 | 3.215 | 3.217 | 3.219 | 3.221 | 3.223 | 3.225 |
| 9.5 | 3.227 | 3.229 | 3.231 | 3.233 | 3.235 | 3.237 | 3.239 | 3.241 | 3.242 | 3.244 |
| 9.6 | 3.246 | 3.248 | 3.250 | 3.252 | 3.254 | 3.256 | 3.258 | 3.260 | 3.262 | 3.264 |
| 9.7 | 3.266 | 3.268 | 3.269 | 3.271 | 3.273 | 3.275 | 3.277 | 3.279 | 3.281 | 3.283 |
| 9.8 | 3.285 | 3.287 | 3.289 | 3.291 | 3.293 | 3.295 | 3.297 | 3.298 | 3.300 | 3.302 |
| 9.9 | 3.304 | 3.305 | 3.307 | 3.309 | 3.311 | 3.313 | 3.316 | 3.318 | 3.320 | 3.321 |

| $\eta_{rel}$ | 0.0 | 0.1 | 0.2 | 0.3 | 0.4 | 0.5 | 0.6 | 0.7 | 0.8 | 0.9 |
|---|---|---|---|---|---|---|---|---|---|---|
| 10 | 3.32 | 3.34 | 3.36 | 3.37 | 3.39 | 3.41 | 3.43 | 3.45 | 3.46 | 3.48 |
| 11 | 3.50 | 3.52 | 3.53 | 3.55 | 3.56 | 3.58 | 3.60 | 3.61 | 3.63 | 3.64 |
| 12 | 3.66 | 3.68 | 3.69 | 3.71 | 3.72 | 3.74 | 3.76 | 3.77 | 3.79 | 3.80 |
| 13 | 3.80 | 3.83 | 3.85 | 3.86 | 3.88 | 3.89 | 3.90 | 3.92 | 3.93 | 3.95 |
| 14 | 3.96 | 3.97 | 3.99 | 4.00 | 4.02 | 4.03 | 4.04 | 4.06 | 4.07 | 4.09 |
| 15 | 4.10 | 4.11 | 4.13 | 4.14 | 4.15 | 4.17 | 4.18 | 4.19 | 4.20 | 4.22 |
| 16 | 4.23 | 4.24 | 4.25 | 4.27 | 4.28 | 4.29 | 4.30 | 4.31 | 4.33 | 4.34 |
| 17 | 4.35 | 4.36 | 4.37 | 4.38 | 4.39 | 4.41 | 4.42 | 4.43 | 4.44 | 4.45 |
| 18 | 4.46 | 4.47 | 4.48 | 4.49 | 4.50 | 4.52 | 4.53 | 4.54 | 4.55 | 4.56 |
| 19 | 4.57 | 4.58 | 4.59 | 4.60 | 4.61 | 4.62 | 4.63 | 4.64 | 4.65 | 4.66 |

The average degree of polymerization of the cellulose powder can be specifically measured by a method described in examples below.

[Average particle diameter $D_{50}$]

The average particle diameter $D_{50}$ of the cellulose powder according to the present embodiment is preferably 15 $\mu$m or greater and 300 $\mu$m or less, more preferably 20 $\mu$m or greater and 200 $\mu$m or less, still more preferably 25 $\mu$m or greater and 150 $\mu$m or less, even still more preferably 25 $\mu$m or greater and 100 $\mu$m or less, and particularly preferably 25 $\mu$m or greater and 70 $\mu$m or less. In a case where the average particle diameter $D_{50}$ of the cellulose powder is greater than or equal to the above-described lower limits, the fluidity tends to be improved. Meanwhile, in a case where the average particle diameter $D_{50}$ of the cellulose powder is less than or equal to the above-described upper limits, the cellulose powder tends to be uniformly mixed with the active component such as a drug.

Further, the average particle diameter $D_{50}$ of the cellulose powder is a particle diameter of a cumulative volume of 50% which is measured by a laser diffraction/scattering-type particle size distribution analyzer (LA-950 V2 type (trade name), manufactured by Horiba, Ltd.).

Specifically, the average particle diameter $D_{50}$ can be measured by the method described in the examples below.

[Water absorption rate Cv]

In the cellulose powder of the present embodiment, the water absorption rate Cv is preferably 2.0 $g^2$/s or greater and 12.0 $g^2$/s or less, more preferably 3.0 $g^2$/s or greater and 10.0 $g^2$/s or less, and still more preferably 4.5 $g^2$/s or greater and 10.0 $g^2$/s or less. In a case where the water absorption rate Cv is in the above-described ranges, high compression moldability can be exhibited.

The water absorption rate Cv of the cellulose powder is measured by the method described below.

First, a Teflon (registered trademark) container is filled with about 5.0 g of cellulose powder that has passed through a sieve having an opening size of 500 $\mu$m, and tapping is performed under the conditions that the number of times of performing tapping is 300 times, the stroke length is 18 mm, and the mass of the weight is 198 g. The cellulose powder after tapping is sufficiently immersed in 300 mL of pure water at a rate of 0.5 mm/s until the cellulose powder enters a saturated state, and the square ($g^2$) of the permeation rate of pure water until the cellulose powder enters the saturated state during the measurement time is measured using a commercially available water absorption rate measuring device (PENETO ANALYZER PNT-N type (trade name), manufactured by Hosokawa Micron Corporation). A graph of the square ($g^2$) of the permeation rate until the cellulose powder enters the saturated state and the measurement time is linearized by the least squares method, and the slope of the linearized graph is defined as the water absorption rate ($g^2$/s).

Specifically, the water absorption rate Cv can be measured by the method described in the examples below.

[Ratio $Cv/D_{50}$ of water absorption rate Cv to average particle diameter $D_{50}$]

$Cv/D_{50}$ can be calculated by dividing the water absorption rate Cv ($g^2$/s) by the average particle diameter $D_{50}$ ($\mu$m).

In the cellulose powder of the present embodiment, the ratio $Cv/D_{50}$ of the water absorption rate Cv ($g^2$/s) to the average particle diameter $D_{50}$ ($\mu$m) is preferably 0.12 $g^2$/(s·$\mu$m) or greater, more preferably 0.13 $g^2$/(s·$\mu$m) or greater, still more preferably 0.14 $g^2$/(s·$\mu$m) or greater, even still more preferably 0.153 $g^2$/(s·$\mu$m) or greater, and particularly preferably 0.154 $g^2$/(s·$\mu$m) or greater. In a case where the ratio $Cv/D_{50}$ is greater than or equal to the above-described lower limits, hydrogen bonds between cellulose particles in the cellulose powder can be strengthened, and plastic deformability can be improved. In this manner, high compression moldability can be exhibited. That is, in a case where the ratio $Cv/D_{50}$ of the cellulose powder of the present embodiment is controlled to be greater than or equal to the above-described lower limits, satisfactory moldability can be ensured regardless of the shape of the particles.

Meanwhile, the upper limit of the ratio $Cv/D_{50}$ is not particularly limited, but can be set to, for example, 0.50 $g^2$/(s·$\mu$m), 0.30 $g^2$/(s·$\mu$m), 0.25 $g^2$/(s·$\mu$m), or 0.24 $g^2$/(s·$\mu$m).

[Loose bulk density]

In the cellulose powder of the present embodiment, the loose bulk density is preferably 0.135 $g/cm^3$ or less and more preferably 0.130 $g/cm^3$ or less. In a case where the loose bulk density is less than or equal to the above-described upper limits, the mechanical strength can be sufficiently imparted to the molded article.

Meanwhile, the lower limit of the loose bulk density is not particularly limited, but can be set to, for example, 0.03 $g/cm^3$, 0.05 $g/cm^3$, 0.07 $g/cm^3$, or 0.08 $g/cm^3$.

The loose bulk density of the cellulose powder is measured by the method described below.

A 25 mL cylindrical metal container is filled with the cellulose powder using a Scott volumeter (model ASTM B-329-85, manufactured by Tsutsui Rikagaku Kikai Co., Ltd.). The cellulose powder contained in the 25 mL cylindrical metal container is scraped off, and the loose bulk density ($g/cm^3$) is calculated by dividing the mass (g) of the cellulose powder contained in the container by 25 mL.

Specifically, the loose bulk density can be measured by the method described in the examples below.

In the cellulose powder of the present embodiment, a relationship between the ratio Y ($Cv/D_{50}$) ($g^2/(s·\mu m)$) of the water absorption rate Cv to the average particle diameter $D_{50}$ and the loose bulk density X ($g/cm^3$) satisfies preferably Expression (1-1) and more preferably Expression (1-2). In a case where the ratio $Cv/D_{50}$ and the loose bulk density are in a range where the above-described relational expression is satisfied, the hydrogen bonds between the cellulose particles in the cellulose powder can be strengthened, and the plastic deformability can be improved. In this manner, high compression moldability can be exhibited. That is, in the cellulose powder of the present embodiment, satisfactory moldability can be ensured regardless of the shape of the particles by controlling the ratio Y ($Cv/D_{50}$) and the loose bulk density (X) to be in a range where the above-described relational expression is satisfied.

$$Y \geq 1.5 \times X - 0.06 \cdots \text{Expression (1-1)}$$

$$Y \geq 2.8 \times X - 0.2 \cdots \text{Expression (1-2)}$$

From the viewpoint of molding a tablet having more excellent moldability and satisfactory hardness and disintegrability, the relationship between the ratio Y ($Cv/D_{50}$) and the loose bulk density X of the cellulose powder of the present embodiment satisfies preferably Expression (1-1) or (1-2) and $X \leq 0.135$, also preferably Expression (1-1) or (1-2) and $0.12 \leq Y$, and more preferably Expression (1-1) or (1-2) and both $X \leq 0.135$ and $0.12 \leq Y$.

In the cellulose powder of the present embodiment, the relationship between the ratio Y ($Cv/D_{50}$) of the water absorption rate Cv to the average particle diameter $D_{50}$ and the loose bulk density X satisfies also preferably Expression (2-1), more preferably Expression (2-2), and still more preferably Expression (2-3). In addition, the relationship between the ratio Y ($Cv/D_{50}$) and the loose bulk density X of the cellulose powder of the present embodiment satisfies preferably any of Expressions (2-1) to (2-3) and $X \leq 0.135$, also preferably any of Expressions (2-1) to (2-3) and $0.12 \leq Y$, and more preferably any of Expressions (2-1) to (2-3) and both $X \leq 0.135$ and $0.12 \leq Y$.

$$Y \leq -2.0 \times X + 0.53 \cdots \text{Expression (2-1)}$$

$$Y \leq -2.0 \times X + 0.50 \cdots \text{Expression (2-2)}$$

$$Y \leq -2.0 \times X + 0.47 \cdots \text{Expression (2-3)}$$

In the cellulose powder of the present embodiment, the relationship between the ratio Y ($Cv/D_{50}$) of the water absorption rate Cv to the average particle diameter $D_{50}$ and the loose bulk density X also satisfies preferably Expression (3-1), more preferably Expression (3-2), and still more preferably Expression (3-3). In addition, the relationship between the ratio Y ($Cv/D_{50}$) and the loose bulk density X of the cellulose powder of the present embodiment satisfies preferably any of Expressions (3-1) to (3-3) and satisfies $X \leq 0.135$, more preferably any of Expressions (3-1) to (3-3) and $0.12 \leq Y$, and still more preferably any of Expressions (3-1) to (3-3) and both of $X \leq 0.135$, and $0.12 \leq Y$.

$$Y \geq -0.72 \times X + 0.15 \cdots \text{Expression (3-1)}$$

$$Y \geq -2.0 \times X + 0.28 \cdots \text{Expression (3-2)}$$

$$Y \geq -4.4 \times X + 0.50 \cdots \text{Expression (3-3)}$$

In the cellulose powder of the present embodiment, the relationship between the ratio Y ($Cv/D_{50}$) of the water absorption rate Cv to the average particle diameter $D_{50}$ and the loose bulk density X satisfies preferably Expression (4-1), more preferably Expression (4-2), still more preferably Expression (4-3), and even still more preferably Expression (4-4). In addition, the relationship between Y ($Cv/D_{50}$) and the loose bulk density X of the cellulose powder of the present embodiment satisfies preferably any of Expressions (4-1) to (4-4) and $X \leq 0.135$, also preferably any of Expressions (4-1) to (4-4) and $0.12 \leq Y$, and more preferably any of Expressions (4-1) to (4-4), $X \leq 0.135$, and $0.12 \leq Y$

$$Y \leq X + 0.25 \cdots\cdots \text{Expression (4-1)}$$

$$Y \leq X + 0.21 \cdots\cdots \text{Expression (4-2)}$$

$$Y \leq X + 0.18 \cdots\cdots \text{Expression (4-3)}$$

$$Y \leq X + 0.16 \cdots\cdots \text{Expression (4-4)}$$

In the cellulose powder of the present embodiment, it is preferable that the relationship between the ratio Y $(Cv/D_{50})$ of the water absorption rate Cv to the average particle diameter $D_{50}$ and the loose bulk density X satisfies two or more relational expressions selected from the group consisting of Expressions (1-1) and (1-2), Expressions (2-1) to (2-3), Expressions (3-1) to (3-3), and Expressions (4-1) to (4-4). In addition, in the cellulose powder of the present embodiment, the relationship between the ratio Y $(Cv/D_{50})$ of the water absorption rate Cv to the average particle diameter $D_{50}$ and the loose bulk density X satisfies preferably two or more relational expressions selected from the group consisting of Expressions (1-1) and (1-2), Expressions (2-1) to (2-3), Expressions (3-1) to (3-3), and Expressions (4-1) to (4-4) and $X \leq 0.135$, also preferably two or more relational expressions selected from the group consisting of Expressions (1-1) and (1-2), Expressions (2-1) to (2-3), Expressions (3-1) to (3-3), and Expressions (4-1) to (4-4) and $0.12 \leq Y$, and more preferably two or more relational expressions selected from the group consisting of Expressions (1-1) and (1-2), Expressions (2-1) to (2-3), Expressions (3-1) to (3-3), and Expressions (4-1) to (4-4) and both $X \leq 0.135$ and $0.12 \leq Y$.

From the viewpoint that a tablet having more excellent moldability and both satisfactory hardness and disintegrability can be molded, the relationship between the ratio Y $(Cv/D_{50})$ and the loose bulk density X of the cellulose powder of the present embodiment satisfies preferably any of Expressions (1-1) and (1-2), $X \leq 0.135$, and $0.12 \leq Y$, more preferably any of Expressions (1-1) and (1-2), $X \leq 0.135$, $0.12 \leq Y$, and any of Expressions (2-1) to (2-3), still more preferably any of Expressions (1-1) to (1-2), $X \leq 0.135$, $0.12 \leq Y$, any of Expressions (2-1) to (2-3), and any of Expressions (3-1) to (3-3), or any of Expressions (1-1) and (1-2), $X \leq 0.135$, $0.12 \leq Y$, any of Expressions (2-1) to (2-3), and any of Expressions (4-1) to (4-4), and even still more preferably any of Expressions (1-1) to (1-2), $X \leq 0.135$, $0.12 \leq Y$, any of Expressions (2-1) to (2-3), any of Expressions (3-1) to (3-3), and any of Expressions (4-1) to (4-4). Among these, the relationship satisfies preferably Expression (1-1), $X \leq 0.135$, $0.12 \leq Y$, and Expression (2-3), more preferably Expression (1-1), $X \leq 0.135$, $0.12 \leq Y$, Expression (2-3), and Expression (3-2), still more preferably Expression (1-1), $X \leq 0.135$, $0.12 \leq Y$, Expression (2-3), and Expression (3-3), or Expression (1-1), $X \leq 0.135$, $0.12 \leq Y$, Expression (2-3), and Expression (4-4), and even still more preferably Expression (1-1), $X \leq 0.135$, $0.12 \leq Y$, Expression (2-3), Expression (3-3), and Expression (4-4).

From the viewpoint that a tablet having more excellent moldability and both satisfactory hardness and disintegrability can be molded, the relationship between the ratio Y $(Cv/D_{50})$ and the loose bulk density X of the cellulose powder of the present embodiment satisfies preferably any of Expressions (1-1) and (1-2), $X \leq 0.135$, and $0.12 \leq Y$, more preferably any of Expressions (1-1) and (1-2), $X \leq 0.135$, $0.12 \leq Y$, and any of Expressions (3-1) to (3-3), still more preferably any of Expressions (1-1) and (1-2), $X \leq 0.135$, $0.12 \leq Y$, any of Expressions (3-1) to (3-3), and any of Expressions (4-1) to (4-4), or any of Expressions (1-1) and (1-2), $X \leq 0.135$, $0.12 \leq Y$, any of Expressions (3-1) to (3-3), and any of Expressions (2-1) to (2-3), and even still more preferably any of Expressions (1-1) to (1-2), $X \leq 0.135$, $0.12 \leq Y$, any of Expressions (2-1) to (2-3), any of Expressions (3-1) to (3-3), and any of Expressions (4-1) to (4-4). Among these, the relationship satisfies preferably Expression (1-1), $X \leq 0.135$, $0.12 \leq Y$, and Expression (3-2), more preferably Expression (1-1), $X \leq 0.135$, $0.12 \leq Y$, and Expression (3-3), still more preferably Expression (1-1), $X \leq 0.135$, $0.12 \leq Y$, Expression (3-3), and Expression (2-3), and even still more preferably Expression (1-1), $X \leq 0.135$, $0.12 \leq Y$, Expression (3-3), Expression (2-3), and Expression (4-4).

From the viewpoint that a tablet having more excellent moldability and both satisfactory hardness and disintegrability can be molded, the relationship between the ratio Y $(Cv/D_{50})$ and the loose bulk density X of the cellulose powder of the present embodiment satisfies preferably any of Expressions (1-1) to (1-2), $X \leq 0.135$, and $0.12 \leq Y$, more preferably any of Expressions (1-1) and (1-2), $X \leq 0.135$, $0.12 \leq Y$, and any of Expressions (4-1) to (4-4), still more preferably any of Expressions (1-1) to (1-2), $X \leq 0.135$, $0.12 \leq Y$, any of Expressions (4-1) to (4-4), and any of Expressions (2-1) to (2-3), or any of Expressions (1-1) and (1-2), $X \leq 0.135$, $0.12 \leq Y$, any of Expressions (4-1) to (4-4), and any of Expressions (3-1) to (3-3), and even still more preferably any of Expressions (1-1) and (1-2), $X \leq 0.135$, $0.12 \leq Y$, any of Expressions (4-1) to (4-4), any of Expressions (2-1) to (2-3), and any of Expressions (3-1) to (3-3). Among these, the relationship satisfies preferably Expression (1-1), $X \leq 0.135$, $0.12 \leq Y$, and Expression (4-4), more preferably Expression (1-1), $X \leq 0.135$, $0.12 \leq Y$, Expression (4-4), and Expression (2-3), or Expression (1-1), $X \leq 0.135$, $0.12 \leq Y$, Expression (4-4), Expression (3-2) or (3-3), still more preferably Expression (1-1), $X \leq 0.135$, $0.12 \leq Y$, Expression (4-4), Expression (2-3), and Expression (3-2) or (3-3), and particularly preferably Expression (1-1), $X \leq 0.135$, $0.12 \leq Y$, Expression (4-4), Expression (2-3), and Expression (3-3).

The relationship between the ratio Y (Cv/D$_{50}$) and the loose bulk density X of the cellulose powder of the present embodiment satisfies preferably any of Expressions (1-1) and (1-2) and any Expressions (2-1) to (2-3), more preferably any of Expressions (1-1) and (1-2), any of Expressions (2-1) to (2-3), and any of Expressions (3-1) to (3-3), or any of Expressions (1-1) and (1-2), any of Expressions (2-1) to (2-3), and any of Expressions (4-1) to (4-4), and still more preferably any of Expressions (1-1) and (1-2), any of Expressions (2-1) to (2-3), any of Expressions (3-1) to (3-3), and any of Expressions (4-1) to (4-4). Among these, the relationship satisfies preferably Expression (1-1) and Expression (2-3), more preferably Expression (1-1), Expression (2-3), and Expression (3-2), still more preferably Expression (1-1), Expression (2-3), and Expression (3-3), or Expression (1-1), Expression (2-3), and Expression (4-4), and even still more preferably Expression (1-1), Expression (2-3), Expression (3-3), and Expression (4-4).

The relationship between the ratio Y (Cv/D$_{50}$) and the loose bulk density X of the cellulose powder of the present embodiment satisfies preferably any of Expressions (1-1) and (1-2) and any of Expressions (3-1) to (3-3), more preferably any of Expressions (1-1) and (1-2), any of Expressions (3-1) to (3-3), and any of Expressions (2-1) to (2-3), or any of Expressions (1-1) and (1-2), any of Expressions (3-1) to (3-3), and any of Expressions (4-1) to (4-4), and still more preferably any of Expressions (1-1) and (1-2), any of Expressions (2-1) to (2-3), any of Expressions (3-1) to (3-3), and any of Expressions (4-1) to (4-4). Among these, the relationship satisfies preferably Expression (1-1) and Expression (3-2), more preferably Expression (1-1) and Expression (3-3), still more preferably Expression (1-1), Expression (3-3), and Expression (2-3), or Expression (1-1), Expression (3-3), and Expression (4-4), and even still more preferably Expression (1-1), Expression (2-3), Expression (3-3), and Expression (4-4).

The relationship between the ratio Y (Cv/D$_{50}$) and the loose bulk density X of the cellulose powder of the present embodiment satisfies preferably any of Expressions (1-1) and (1-2) and any of Expressions (4-1) to (4-4), more preferably Expression (1-1) and Expression (4-1), still more preferably Expression (1-1) and Expression (4-2), even still more preferably Expression (1-1) and Expression (4-3), and particularly preferably Expression (1-1) and Expression (4-4).

[Cohesive force F]

In the cellulose powder of the present embodiment, the cohesive force F is preferably 30 N% or greater and 100 N% or less, more preferably 45 N% or greater and 90 N% or less, still more preferably 50 N% or greater and 80 N% or less, and even still more preferably 55 N% or greater and 80 N% or less. In a case where the cohesive force F is in the above-described ranges, the cohesive force of the cellulose powder is increased, and a molded article having a low abrasion degree is obtained in the step of producing a molded article.

The cohesive force F of the cellulose powder is measured by the method described below.

First, a shear cell ($\varphi$15 mm, upper and lower cell lengths: 34 mm, 5 mm) is filled with powder of the cellulose powder, and the upper surface of the powder layer is flattened. Thereafter, a shearing test is performed at a shearing rate of 50 $\mu$m/sec with a target indentation load of 20 N as a condition for indentation control using a powder layer shearing force measuring device (NS-S300 type (trade name), manufactured by Nano Seeds Corporation). In a case where the load reaches the target load, the indentation is stopped, the lateral sliding is started, and the static friction coefficient and the dynamic friction coefficient are measured. In the analysis, the elapsed time (s) of the measurement is plotted on the horizontal axis and the load (N) applied to the shear surface is plotted on the vertical axis, the maximum value and the minimum value of the shear surface load are determined, and the cohesive force defined by the following equation is calculated.

[Cohesive force F (N%)] = ([Maximum value of shear surface Load (N)] - [minimum value of shear surface load (N)])/ ([maximum value of shear surface load (N)] $\times$ 100)

The cohesive force F of the cellulose powder can be specifically measured by a method described in examples below.

[Ratio F/D$_{50}$ of cohesive force F to average particle diameter D$_{50}$]

In the cellulose powder of the present embodiment, a ratio F/D$_{50}$ of the cohesive force F to the average particle diameter D$_{50}$ is preferably 0.01 N%/$\mu$m or greater, more preferably 0.8N%/$\mu$m or greater, still more preferably 0.9N%/$\mu$m or greater, even still more preferably 1.3N%/$\mu$m or greater, particularly preferably 1.4N%/$\mu$m or greater, and most preferably 1.6N%/$\mu$m or greater. In a case where the ratio F/D$_{50}$ is greater than or equal to the above-described lower limits, the cohesive force of the cellulose powder is increased, and a molded article having a low abrasion degree is obtained in the step of producing a molded article. The F/D$_{50}$ can be set to the above-described lower limits or greater by controlling the shape of the cellulose particles in the cellulose powder.

Meanwhile, the upper limit of the ratio $F/D_{50}$ is not particularly limited, but can be set to 3.0 N%/$\mu$m, 2.5 N%/$\mu$m, 2.4 N%/$\mu$m, 2.3 N%/$\mu$m, or 2.2 N%/$\mu$m.

[Powder dynamic friction angle]

In the cellulose powder of the present embodiment, the powder dynamic friction angle is preferably 30° or greater, more preferably 40° or greater, still more preferably 43° or greater, even still more preferably 49° or greater, particularly preferably 50° or greater, and most preferably 51° or greater. In a case where the powder dynamic friction angle is greater than or equal to the above-described lower limit, the friction between the cellulose powders is increased, and a molded article having a low abrasion degree is obtained in the step of producing a molded article.

Meanwhile, the upper limit of the powder dynamic friction angle is not particularly limited, but can be set to 70°, 65°, 60°, or 57°.

The powder dynamic friction angle of the cellulose powder is measured by the method described below.

First, a shear cell ($\varphi$15 mm, upper and lower cell lengths: 34 mm, 5 mm) is filled with powder of the cellulose powder, and the upper surface of the powder layer is flattened. Thereafter, a shearing test is performed at a shearing rate of 50 $\mu$m/sec with a target indentation load of 20 N, 30 N, and 40 N as a condition for indentation control using a powder layer shearing force measuring device (NS-S300 type (trade name), manufactured by Nano Seeds Corporation). In a case where the load reaches the target load, the indentation is stopped, the lateral sliding is started, the static friction coefficient and the dynamic friction coefficient are measured. In the analysis, the elapsed time (s) of the measurement is plotted on the horizontal axis and the shearing force (N) is plotted on the vertical axis, and the maximum value of the shearing force and the indentation load value in this case are determined. The result is plotted on a graph in which the vertical axis is the shear stress and the horizontal axis is the normal stress, and the angle of the straight line connecting the origin and these points with respect to the X-axis is calculated as the powder dynamic friction angle.

[Compression ratio]

**In** the cellulose powder of the present embodiment, the compression ratio is preferably 40% or greater, more preferably 45% or greater, still more preferably 50% or greater, even still more preferably 53% or greater, and particularly preferably 55% or greater. In a case where the compression ratio is greater than or equal to the above-described lower limits, cellulose powder that is easily compressed is obtained, and high compression moldability can be exhibited.

Meanwhile, the upper limit of the compression ratio is not particularly limited, but can be set to 70%, 65%, or 62%.

The compression ratio of the cellulose powder is measured by the method described below.

First, the packed bulk density is measured with a powder property evaluation device (powder tester, manufactured by HOSOKAWA MICRON CORPORATION). In this case, a sieve having an opening size of 710 $\mu$m and a metal funnel having an inner diameter of 0.8 cm is used, and the VIBRATION is set to 2.0 (supply source: AC 100 V, 60 Hz). The loose bulk density is measured by the method described above, and the compression ratio defined by the following equation is calculated.

[Compression ratio (%)] = ([Packed bulk density (g/cm$^3$)] - [loose bulk density (g/cm$^3$)])/[packed bulk density (g/cm$^3$)] $\times$ 100

[Structure of particles]

As the cellulose powder of the present embodiment, cellulose powder in which the proportion of particles having a primary particle structure is large is preferable. Further, the structure formed by aggregation of the primary particles is referred to as a secondary aggregate structure. Whether the particles are primary particles or have a secondary aggregate structure can be determined by imaging the particles at a magnification of 5 times with a dry image analysis device (Malvern Morphorogi G3S), and it is possible to confirm whether the particles are primary particles or have a secondary aggregate structure in which the boundary of the primary particles is clear from the obtained image. In the cellulose powder of the present embodiment, the effect of exhibiting high moldability and preventing abrasion is verified for the first time by focusing on the shape of the cellulose primary particles and controlling the proportion of particles having a primary particle structure to be in a specific range. The primary particle structure is closely related to the moldability of the molded article. As the number of primary particles increases, the plastic deformability during compression increases, the entanglement of particles is likely to occur, and thus high moldability and the effect of preventing abrasion can be exhibited.

The proportion (primary particle ratio) of particles having a primary particle structure in the total number of particles of the cellulose powder can be measured by the method described below.

10,000 particles are imaged and subjected to image analysis to determine the particle diameter and the aspect ratio of each particle equivalent to the area of the circle using a dry image analysis device (Malvern Morphorogi G3S). As a result, particle images in which the particle diameter equivalent to the area of the circle is in a range of 30 μm to 90 μm and the aspect ratio is the average value of the aspect ratios of 10,000 particles + 0.1 or less are extracted. The particle shape in each of the extracted particle images is visually confirmed, the single particle is determined as a primary particle, and the particles that are aggregated are determined as secondary particles, and the proportion of the particle images determined as the primary particles in the extracted particle images is calculated as the primary particle ratio. Further, the particle shape in the particle image may be confirmed using image processing software.

As a simple method, particles in which a brightness dispersion value of each of the extracted particle images is selected from a range of 1 to 26 are determined as primary particles, and particles in which the brightness dispersion value thereof is selected from a range of 26 to 100 are determined as secondary particles, and the primary particle ratio (%) can also be calculated by "number of primary particles/total number of particles × 100". **In** the present example, the method using the above-described image processing software is employed.

**In** the present invention and the specification of the present application, the cellulose powder in which 20% or greater of the total number of particles have a primary particle structure, that is, the cellulose powder in which the primary particle ratio is 20% or greater is referred to as "cellulose powder having a large proportion of particles having a primary particle structure". Further, in the cellulose powder of the present embodiment, the proportion of the particles having a primary particle structure in the total number of particles is preferably 25% or greater, more preferably 26% or greater, still more preferably 30% or greater, and even still more preferably 31% or greater. Meanwhile, the upper limit of the primary particle ratio is not particularly limited, but can be set to 50% or 45%.

The cellulose powder of the present embodiment exhibits high moldability since the proportion of particles having a primary particle structure is large and the particles are likely to be entangled. The amount of the cellulose powder to be added for preparing a molded article can be reduced by using the cellulose powder of the present embodiment due to this property, and a smaller molded article can be obtained without changing the amount of the active component such as a drug.

In addition, since the powder having a primary particle structure can be molded with a low compression force during molding, the proportion of particles having a primary particle structure in the powder is closely related not only to the moldability and the abrasion degree but also to the disintegrability of the molded article and the elution properties of the active component. In a case where the compression force during molding is low, the gap between the primary particles is maintained in the molded article. That is, a molded article having a large number of cellulose primary particle gaps is obtained by using the cellulose powder of the present embodiment in which the proportion of particles having a primary particle structure is large.

In the molded article containing a large number of cellulose primary particle gaps, the permeation of water between the primary particles is fast in water, and the particles are promoted to collapse. In addition, in a case where the active component is maintained in the cellulose primary particle gaps, the contact between the active component and water is also promoted, and the elution of the active component is also promoted. That is, an effect of improving the disintegrability of the molded article and the elution properties of the active component can be obtained by producing a molded article using the cellulose powder of the present embodiment.

In addition, the cellulose primary particles have high compression moldability and an effect of preventing abrasion, and are likely to be dispersed in water. Therefore, a molded article having an excellent balance between moldability and disintegrability can be obtained by using the cellulose powder of the present embodiment.

In addition, the cellulose primary particle gaps in the molded article also contribute to the maintenance of the liquid component. Therefore, particularly, the liquid component can be prevented from effusion by producing a molded article using the cellulose powder of the present embodiment.

In addition, the cellulose primary particle gaps in the molded article also contribute to the improvement of the mixing speed with other components such as the active component. Therefore, the mixing uniformity is improved, and the variation in concentration can be significantly reduced by producing a molded article using the cellulose powder of the present embodiment.

Further, since the cellulose powder of the present embodiment has a large proportion of particles having a primary particle structure, the loose bulk density is small, the cellulose powder is light, and thus the dynamic fluidity is high. Therefore, the variation in the mass of the molded article can be reduced by using the cellulose powder of the present embodiment.

[L/D of primary particles]

In the cellulose powder of the present embodiment, a ratio L/D of a major axis (L) to a minor axis (D) of the primary particles is preferably 2.0 or greater, more preferably 2.5 or greater, still more preferably 3.0 or greater, even still more preferably 3.5 or greater, and particularly preferably 3.8 or greater. **In** a case where the ratio L/D of the primary particles is greater than or equal to the above-described lower limits, entanglement between the elongated particles can be promoted during compression molding of the cellulose powder, and thus high compression moldability can be exhibited.

Meanwhile, the upper limit of the ratio L/D of the primary particles of the cellulose powder of the present embodiment is not particularly limited, but can be set to 6.0 or 5.0.

The ratio L/D of the primary particles of the cellulose powder is measured by the method described below.

Particle images of the primary particles are extracted by the same method as described above using a dry image analysis device (Malvern Morphorogi G3S). The ratio of major axis (L)/minor axis (D) is calculated from the major axis (L) and the minor axis (D) obtained from each of the extracted particle images, and the average value of the ratios of all the extracted particle images of the primary particles is calculated as the ratio L/D of the primary particles of the cellulose powder.

[Method of producing cellulose powder]

A method of producing the cellulose powder of the present embodiment will be described below.

In the production of the cellulose powder according to the present embodiment, for example, first, a dispersion liquid (hereinafter, also referred to as "cellulose dispersion liquid") containing a natural cellulose-based substance in which the average particle diameter of the cellulose primary particles is 10 $\mu$m or greater and less than 300 $\mu$m, the average width thereof is 2 $\mu$m or greater and 30 $\mu$m or less, and the average thickness thereof is 0.5 $\mu$m or greater and 5 $\mu$m or less is obtained. By forming the cellulose primary particles in the cellulose dispersion liquid into such a shape, the entanglement of the primary particles of the cellulose powder can be promoted in a case of compression-molding the cellulose powder after drying.

In the related art, as the number of primary particles in the cellulose dispersion liquid increases, the entanglement of particles in the drying step is likely to occur, and the particles of the cellulose powder form a secondary aggregate structure, which makes it difficult to maintain the primary particle structure.

Meanwhile, in the cellulose powder of the present embodiment, it has been verified for the first time that, high plastic deformability of the cellulose primary particles and the entanglement of the primary particles can be promoted in a case of compression-molding the cellulose powder by focusing on the cellulose primary particle structure and controlling the cellulose primary particle structure to be in a specific range. By promoting the high plastic deformability of the cellulose primary particles and the entanglement of the primary particles, the moldability is more likely to be imparted to the molded article as compared with the related art, and a smaller molded article can be obtained for the first time without changing the amount of the active component such as a drug.

In consideration of the above-described circumstances, the production of the cellulose powder according to the present embodiment, from the viewpoint that the secondary aggregate structure is more likely to be obtained as the number of primary particles in the cellulose dispersion liquid increases, it is preferable to prepare a cellulose dispersion liquid having a cellulose concentration of 0.5% by mass or greater and 40% by mass or less.

Next, the obtained cellulose dispersion liquid is dried to obtain the cellulose powder of the present embodiment.

The natural cellulose-based substance may be of a plant origin or an animal origin. Examples of the natural cellulose-based substance include fibrous substances derived from natural products containing cellulose, such as wood, bamboo, wheat straw, rice straw, cotton, ramie, bagasse, kenaf, beet, sea squirt, and bacterial cellulose. The natural cellulose-based substance has a cellulose type I crystal structure. As a raw material, one kind of the above-described natural cellulose-based substance or a mixture of two or more kinds thereof may be used.

In addition, it is preferable to use the natural cellulose-based substance in the form of purified pulp. A method of purifying the pulp is not particularly limited, and any pulp such as dissolving pulp, kraft pulp, or NBKP pulp may be used. From the viewpoint of high $\alpha$-cellulose purity, availability, and high supply stability, pulp derived from wood is preferable.

Among these, as the wood pulp, wood pulp having a level-off degree of polymerization of 130 or greater and 250 or less which is measured by a copper ethylenediamine solution method, a whiteness of 90% or greater and 99% or less, S10 of 5% or greater and 20% or less, and S18 of 1% or greater and 10% or less is preferable. In a case where the level-off degree of polymerization is greater than or equal to the above-described lower limit, moldability is more likely to be exhibited. Meanwhile, in a case where the degree of polymerization is less than or equal to the above-described upper limit, the average width and the average thickness of the cellulose primary particles can be easily controlled to be in specific ranges. In a case where the whiteness is greater than or equal to the above-described lower limit, the appearance of the cellulose powder can be further enhanced. The whiteness is as high as possible, but the whiteness is usually about 99% at most. In a case where S10 and S18 are in the above-described ranges, the moldability and the yield are further enhanced. Here, in the natural cellulose-based substance, a raw material such as pulp may or may not be hydrolyzed. In particular, in a case of hydrolysis, the hydrolysis may be acid hydrolysis, alkaline oxidative decomposition, hot water decomposition, or steam explosion. Among these hydrolysis methods, any one method may be used alone, or two or more methods may be used in combination.

In the above-described production method, water is preferable as the medium used in a case where the solid content including the natural cellulose-based substance is subsequently dispersed in an appropriate medium. In addition, the medium may be a medium other than water and is not particularly limited as long as the medium is used industrially, and for example, a mixture of water and an organic solvent may be used. Examples of the organic solvent include alcohols such as methanol, ethanol, isopropyl alcohol, butyl alcohol, 2-methylbutyl alcohol, and benzyl alcohol, hydrocarbons such as pentane, hexane, heptane, and cyclohexane, and ketones such as acetone and ethyl methyl ketone. In particular, the organic solvent is preferably an organic solvent used for a pharmaceutical product, and examples thereof include those classified as solvents in "Encyclopedia of Pharmaceutical Excipients" (published by Yakuji Nippo, Ltd.). Water or an organic solvent may be used alone or two or more kinds thereof may be used in combination, or the solid content may be temporarily dispersed in one medium and then dispersed in a different medium after the medium is removed.

In the method of producing a cellulose powder according to the present embodiment, the natural cellulose-based substance is subjected to a treatment using a known method of performing a mechanical treatment such as pulverization or grinding, performing a chemical treatment such as hydrolysis, appropriately combining both treatments, or the like. By performing the treatment, a cellulose dispersion liquid in which the average particle diameter of the cellulose primary particles is 10 $\mu$m or greater and less than 300 $\mu$m, the average width thereof is 2 $\mu$m or greater and 30 $\mu$m or less, the average thickness thereof is 0.5 $\mu$m or greater and 5 $\mu$m or less, and the solid content thereof is 0.5% by mass or greater and 40% by mass or less is prepared. Next, the cellulose dispersion liquid is dried.

In a case of fibers constituting the natural cellulose-based substance, or in a case where the natural cellulose-based substance is subjected to a mechanical treatment such as pulverization or grinding, or the natural cellulose-based substance is subjected to a chemical treatment such as hydrolysis, the cellulose primary particles in the specification of the present application refer to particles having a size in a range of 1 $\mu$m or greater and 500 $\mu$m or less, which are newly formed by dividing the fibers.

Examples of a method of adjusting the average particle diameter of the cellulose primary particles to less than 300 $\mu$m include a mechanical treatment such as pulverization or grinding, a known separation treatment such as cyclone, centrifugation, or sieving, and a method of appropriately combining both treatments. The average particle diameter of the cellulose primary particles can be adjusted to less than 300 $\mu$m by appropriately adjusting the conditions that are known to usually affect the treatment, such as the treatment amount, the shearing force (affected by the rotation speed, the shape of the blade, the blade dimensions, and the like), the centrifugal force, and the sieve opening during the treatment. In addition, for example, in a case where a chemical treatment such as acid hydrolysis is performed, the average particle diameter of the cellulose primary particles can be adjusted to less than 300 $\mu$m by appropriately changing the conditions such as the acid concentration and the temperature during the chemical treatment or by appropriately changing the conditions that are already known to affect the mechanical treatment, the separation treatment, and the like, in addition to the above-described conditions.

In a case where hydrolysis is carried out, in general, as the concentration of an acid or an alkali in a hydrolysis solution and the reaction temperature are increased, the degree of polymerization of cellulose tends to decrease, and the average particle diameter of cellulose particles in a dispersion liquid tends to decrease. In addition, even in a case where the stirring force of the solution is increased, the average particle diameter of the cellulose particles in the dispersion liquid tends to decrease. Therefore, the degree of polymerization of the cellulose particles and the average particle diameter thereof can be controlled to be in a desired range by adjusting the degree of polymerization of the raw material cellulose and the stirring force in the hydrolysis or the dispersion step of the natural cellulose-based substance. The stirring force depends on the width, the height, and the volume of a stirring layer, the kind of blade, the blade diameter, the stirring rotation speed, and the like. Therefore, although the product of the blade diameter (m) and the stirring rotation speed (rpm) is difficult to define to a specific range, but is preferably 5 or greater and 2,000 or less, more preferably 10 or greater and 1,000 or less, and still more preferably 10 or greater and 700 or less.

In the method of producing cellulose powder according to the present embodiment, in the hydrolysis of the raw material cellulose, it is preferable that the reaction temperature is set to be relatively high while the concentration of an acid or an alkali is set to be relatively low, and the hydrolysis is performed in a relatively short time. By drying the cellulose dispersion liquid prepared under such reaction conditions, cellulose powder in which the ratio ($Cv/D_{50}$) is 0.12 g$^2$/(s·$\mu$m) or greater and the loose bulk density is 0.135 g/cm$^3$ or less is likely to be obtained. For example, hydrolysis can be carried out in a hydrochloric acid aqueous solution having a concentration of 0.05% by mass or greater and 0.3% by mass or less, preferably 0.1% by mass or greater and 0.3% by mass or less, and more preferably 0.1% by mass or greater and 0.2% by mass or less at 85°C or higher and 150°C or lower, preferably 100°C or higher and 135°C or lower, and more preferably 105°C or higher and 130°C or lower. The reaction time of the hydrolysis reaction is appropriately set depending on the amount of the raw material cellulose, the amount of the reaction solution, the strength of the stirring force of the solution, and the like. For example, the time is preferably 10 minutes or longer and 3 hours or shorter, more preferably 30 minutes or longer and 3 hours or shorter, and still more preferably 1 hour or longer and 2 hours or shorter.

A method of setting the average width of the cellulose primary particles to 2 $\mu$m or greater and 30 $\mu$m or less and the average thickness to 0.5 $\mu$m or greater and 5 $\mu$m or less is not particularly limited as long as the method is a method of mainly tearing the cellulose primary particles in the longitudinal direction. Specific examples of such a method include a method in which wood pulp is subjected to a treatment such as a high-pressure homogenization treatment, and as necessary, a mechanical treatment such as grinding or a separation treatment, or a combination of both treatments. In a case of performing the high-pressure homogenization treatment, the pressure may be appropriately adjusted to be in a range of about 10 MPa or greater and 200 MPa or less depending on the treatment amount. In addition, for example, pulp in which the average width of the cellulose primary particles is 2 μm or greater and 30 μm or less and the average thickness thereof is 0.5 μm or greater and 5 μm or less may be selected and used.

The content of the cellulose dispersion liquid is preferably 0.5% by mass or greater and 40% by mass or less. By obtaining a cellulose dispersion liquid satisfying the above-described conditions, the particles of the cellulose powder can be maintained to have a primary particle structure. Therefore, the plastic deformability during compression can be increased, and the entanglement between the primary particles can be further promoted.

The cellulose concentration in the cellulose dispersion liquid is measured by the method described below.

5 g of the cellulose dispersion liquid is placed on an aluminum sheet installed in an infrared moisture meter (model FD-240, manufactured by Kett Electric Laboratory Co., Ltd.) and heated and dried by irradiation with infrared rays. The cellulose concentration in the cellulose dispersion liquid is calculated by the following equation using the moisture value (%) obtained from a change in mass due to the evaporation of the moisture.

[Cellulose concentration (%) in cellulose dispersion liquid] = 100 - [moisture value (%)]

The primary particle structure is likely to be maintained in a case of drying the cellulose dispersion liquid by preparing a cellulose dispersion liquid in which the average particle diameter of the cellulose primary particles is 10 μm or greater and less than 300 μm, the average width thereof is 2 μm or greater and 30 μm or less, and the average thickness thereof is 0.5 μm or greater and 5 μm or less and preferably a cellulose dispersion liquid in which the cellulose concentration in the cellulose dispersion liquid is 0.5% by mass or greater and 40% by mass or less in addition to the above-described conditions. Further, since the cellulose primary particles having a specific average particle diameter, a specific average width, and a specific average thickness are likely to be bent, in a case of compression-molding the cellulose powder, a molded article that promotes high plastic deformability of the cellulose primary particles and the entanglement of the primary particles and exhibits high moldability and an effect of preventing abrasion can be obtained.

In a case where the average particle diameter of the cellulose primary particles is less than the above-described upper limit, and the shape of the cellulose primary particles is in a specific range, the cellulose primary particles are more likely to be bent, the plastic deformability during compression molding can be further increased, and the entanglement between the primary particles can be further promoted. Meanwhile, in a case where the average particle diameter of the cellulose primary particles is greater than or equal to the above-described lower limit, the formation of fine particles can be prevented, the cellulose primary particle gaps in the molded article can be more effectively maintained, and a molded article having more excellent disintegrability and elution properties can be obtained.

In a case where the average width of the cellulose primary particles is less than the above-described upper limit, the cellulose primary particles are more likely to be bent, the plastic deformability during compression molding can be further increased, and the entanglement between the primary particles can be further promoted. Meanwhile, in a case where the average width of the cellulose primary particles is greater than or equal to the above-described lower limit, the cellulose primary particle gaps in the molded article can be more effectively maintained, and a molded article having more excellent disintegrability and elution properties can be obtained.

In a case where the average thickness of the cellulose primary particles is less than the above-described upper limit, the cellulose primary particles are more likely to be bent, the plastic deformability during compression molding can be further increased, and the entanglement between the primary particles can be further promoted. It is preferable that the lower limit of the average thickness of the cellulose primary particles decreases from the viewpoint that the particles are likely to be entangled, but the average thickness thereof is usually about 0.5 μm.

In a case where the average width of the cellulose primary particles is greater than or equal to the above-described lower limit and the average thickness thereof is greater than or equal to the above-described lower limit, the formation of fine particles is prevented, the cellulose primary particle gaps in the molded article can be more effectively maintained, and the disintegrability and the elution properties are more excellent.

The method of producing the cellulose dispersion liquid in the specification of the present application is not particularly limited, and examples thereof include i) a method in which cellulose primary particles obtained by treating a single or two or more kinds of natural cellulose-based substances are used as a cellulose dispersion liquid, ii) a method in which the cellulose dispersion liquid obtained in i) is divided, separately treated, and then mixed to obtain a cellulose dispersion liquid, iii) a method in which the cellulose dispersion liquid obtained in i) or ii) is fractionated, treated, and then mixed to obtain a cellulose dispersion liquid, and iv) a method in which two or more kinds of cellulose primary particles prepared separately are mixed to obtain a cellulose dispersion liquid. Among these, the method i) is preferable from the economic viewpoint. The treatment method performed here may be a wet method or a dry method, and the materials obtained by the wet method may be mixed before drying, the materials obtained by the dry method may be mixed before drying, or the materials obtained by the wet method or the dry method may be used in combination.

**In** addition, the method performed on the cellulose dispersion liquid is not particularly limited as long as the method is a known method, and examples thereof include a mechanical treatment such as pulverization or grinding, centrifugation using a cyclone or a centrifuge, and separation treatment such as classification using a sieve. These treatment methods may be used alone or in combination of two or more kinds thereof.

Examples of the pulverization method include a screen pulverization method such as a screen mill or a hammer mill, a blade rotation shear screen pulverization method such as a flash mill, an air flow pulverization method such as a jet mill, a ball pulverization method such as a ball mill or a vibration ball mill, and a blade stirring pulverization method.

Examples of the grinding method include a grinding method using a stirring blade such as a one-directional rotation type, a multi-axis rotation type, a reciprocating type, an up-down movement type, a rotation + up-down movement type, or a pipe line type of a portable mixer, a three-dimensional mixer, or a side surface mixer, a jet stream type stirring and grinding method such as a line mixer, a grinding method using a highshear homogenizer, a high-pressure homogenizer, an ultrasonic homogenizer, or the like, and an axis rotation extrusion type grinding method such as a kneader.

The concentration of the cellulose-dispersed particles obtained by the above-described operation in the dispersion liquid before drying is preferably 0.5% by mass or greater 40% by mass or less, more preferably 1.0% by mass or greater and 30% by mass or less, still more preferably 2.0% by mass or greater and 10% by mass or less, particularly preferably 3.0% by mass or greater and 9.0% by mass or less, more particularly preferably 3.5% by mass or greater and 8.0% by mass or less, and most preferably 4.0% by mass or greater and 7.0% by mass or less. In a case where the concentration of the cellulose-dispersed particles in the dispersion liquid is greater than or equal to the above-described lower limits, the average particle diameter of the cellulose particles to be obtained is further increased, and the fluidity is more excellent. Meanwhile, in a case where the concentration of the cellulose-dispersed particles in the dispersion liquid is less than or equal to the above-described upper limits, the apparent specific volume of the cellulose particles is further increased, and the compression moldability is more excellent.

The drying method is not particularly limited, and examples thereof include freeze drying, spray drying, drum drying, shelf drying, air flow drying, and vacuum drying. These drying methods may be used alone or in combination of two or more kinds thereof. Examples of the spraying method in a case of spray drying include a disk type method, a pressurized nozzle method, a pressurized two-fluid nozzle method, and a pressurized four-fluid nozzle method. These spraying methods may be used alone or in combination of two or more thereof. Among these, from the economic viewpoint, spray drying or air flow drying is preferable as the drying method.

In the spray drying, a trace amount of a water-soluble polymer or a surfactant may be added for the purpose of reducing the surface tension of the dispersion liquid, or a foaming agent or a gas may be added to the dispersion liquid for the purpose of promoting the vaporization rate of the medium.

Examples of the water-soluble polymer include water-soluble polymers described in "Encyclopedia of Pharmaceutical Excipients" (published by Yakuji Nippo, Ltd.), such as hydroxypropyl cellulose, hydroxypropyl methyl cellulose, polyacrylic acid, a carboxyvinyl polymer, polyethylene glycol, polyvinyl alcohol, polyvinylpyrrolidone, methyl cellulose, gum arabic, and starch paste. These water-soluble polymers may be used alone or in combination of two or more kinds thereof.

Examples of the surfactant include those classified as surfactants in "Encyclopedia of Pharmaceutical Excipients" (published by Yakuji Nippo, Ltd.), such as phospholipids, glycerin fatty acid esters, polyethylene glycol fatty acid esters, sorbitan fatty acid esters, polyoxyethylene hydrogenated castor oil, polyoxyethylene cetyl ether, polyoxyethylene stearyl ether, polyoxyethylene nonylphenyl ether, polyoxyethylene polyoxypropylene glycol, polyoxyethylene sorbitan monolaurate, polysorbate, sorbitan monooleate, glycerol monostearate, monooxyethylene sorbitan monopalmitate, monooxyethylene sorbitan monostearate, monooxyethylene sorbitan monooleate, sorbitan monopalmitate, and sodium lauryl sulfate. These surfactants may be used alone or in combination of two or more kinds thereof.

Examples of the foaming agent include foaming agents described in "Encyclopedia of Pharmaceutical Excipients" (published by Yakuji Nippo, Ltd.) such as tartaric acid, sodium hydrogen carbonate, potato starch, citric anhydride, medicinal soap, sodium lauryl sulfate, diethanolamide laurate, and lauromacrogol. These foaming agents may be used alone or in combination of two or more kinds thereof.

Further, in addition to the pharmaceutical additive, for example, bicarbonates that generate gas by thermal decomposition, such as sodium bicarbonate and ammonium bicarbonate, and carbonates that generate gas by reacting with an acid, such as sodium carbonate and ammonium carbonate, may be used. Here, in a case where the above-described carbonates are used, the carbonates are required to be used with an acid. Examples of the acid include acid substances, for example, organic acids such as citric acid, acetic acid, ascorbic acid, and adipic acid, protonic acids such as hydrochloric acid, sulfuric acid, phosphoric acid, and nitric acid, and Lewis acids such as boron fluoride. Among these, an acid used as a pharmaceutical product or food is preferable as the acid, but the same effect can be obtained even in a case of using an acid other than the acids described above.

Alternatively, instead of using the foaming agent, a dispersion liquid may be impregnated with gases such as nitrogen, carbon dioxide, liquefied petroleum gas, and dimethyl ether.

These substances that generate gas, such as water-soluble polymers, surfactants, and foaming agents, may be added before drying, and the timing of the addition is not particularly limited.

<Applications>

In a case where the cellulose powder of the present embodiment is blended with a solid preparation containing an active component, a smaller solid preparation having excellent hardness and a low abrasion degree while reducing the variation in mass and maintaining satisfactory disintegrability can be obtained without changing the amount of the active component. Therefore, the cellulose powder is particularly useful as an excipient for a solid preparation aimed at size reduction.

In a case where the cellulose powder of the present embodiment is blended with a solid preparation containing an active component having poor water solubility, the variation in mass is small, and tableting properties are extremely satisfactory while the disintegrability is satisfactorily maintained. Therefore, the cellulose powder is particularly useful as an excipient for a solid preparation containing an active component having poor water solubility.

In addition, in a case where the cellulose powder of the present embodiment is blended with a solid preparation containing a liquid active component or a semi-solid active component, effusion of the liquid active component or the semi-solid active component is prevented while the disintegrability is satisfactorily maintained. Therefore, the cellulose powder is particularly useful as an excipient for a solid preparation containing a liquid active component or a semi-solid active component.

In addition, the cellulose powder of the present embodiment has a trace amount of the active component, and in particular, in a case where the active component having a small average particle diameter $D_{50}$ and high adhesion and aggregation properties is mixed with components other than the active component or blended with a solid preparation, the mixing speed of the active component and the reduction of the concentration variation can be contributed, and the tableting properties are improved. Therefore, the cellulose powder is particularly useful as an excipient for a mixture of a trace amount of an active component particularly having a small average particle diameter $D_{50}$ and high adhesion and aggregation properties with a component other than the active component and an excipient for a solid preparation obtained by using such a mixture.

Further, in a case where the cellulose powder of the present embodiment is blended with a solid preparation containing a sublimable active component, the recrystallization of the sublimable active component due to sublimation can be prevented, and a decrease in product value can be prevented. Therefore, the cellulose powder is particularly useful as an excipient for a solid preparation containing a sublimable active component.

<Molded article>

The molded article according to the present embodiment contains one or more active components and the cellulose powder according to the present embodiment.

In a case where the molded article according to the present embodiment is a tablet, a smaller tablet having excellent hardness and a low abrasion degree while reducing the variation in mass and maintaining satisfactory disintegrability can be obtained without changing the amount of the active component such as a drug by allowing the molded article to contain the cellulose powder of the present embodiment.

In the molded article according to the present embodiment, the contents of the active component and the cellulose powder are not particularly limited, but the content of the active component is 0.001% by mass or greater and 99% by mass or less, and the content of the cellulose powder according to the present embodiment is 1% by mass or greater and 99% by mass or less with respect to the total mass of the molded article, as normal use ranges. In a case where the content of the active component is greater than or equal to the above-described lower limits, the amount effective for curing can be ensured. Meanwhile, in a case where the content of the active component is less than or equal to the above-described upper limits, the content of the cellulose powder of the present embodiment can be set to be greater than or equal to the above-described lower limits, and a molded article exhibiting practical hardness, an abrasion degree, and disintegrability can be obtained.

Further, the molded article according to the present embodiment is obtained by processing the active component and the cellulose powder by a known method such as mixing, stirring, granulation, particle size adjustment, and tableting.

In addition, the molded article according to the present embodiment can also contain, as necessary, an excipient, a disintegrating agent, a binding agent, a fluidizing agent, a lubricant, a flavoring agent, a fragrance, a colorant, and a sweetener, in addition to the active component and the cellulose powder.

Examples of the molded article according to the present embodiment include a tablet, a powder, a fine granule, a granule, an extract, and a pill in a case of being used for a pharmaceutical product. Among these, a tablet is preferable.

In addition, the molded article according to the present embodiment is not limited to a pharmaceutical product, and can be used for food such as confectionery, health food, a food texture improver, or a dietary fiber reinforcing agent, a solid foundation, a bath agent, animal medicine, diagnostic medicine, an agricultural chemical, a fertilizer, or a ceramic catalyst.

[Active component]

The active component includes a pharmaceutically active component, an agricultural chemical component, a fertilizer component, a feed component, a food component, a cosmetic component, a coloring agent, a fragrance, a metal, a ceramic, a catalyst, and a surfactant. In addition, the active component may be in any form such as a solid form (powdery, crystalline, or the like), an oily form, a liquid form, or a semi-solid form. In addition, the coating may be applied for the purpose of controlling elution, reducing bitterness, or the like. The active components may be used alone or in combination of a plurality of kinds thereof. The active component may be used by being dissolved, suspended, or emulsified in a medium.

Among these, a pharmaceutically active component or an active component for food is preferable as the active component.

Examples of the pharmaceutically active component include components that are orally administered, such as an antipyretic analgesic anti-inflammatory drugs, hypnotic and sedative drugs, anti-drowsiness drugs, antivertigo drugs, pediatric analgesic drugs, stomachics, antacids, digestive drugs, cardiac stimulants, antiarrhythmic drugs, antihypertensive drugs, vasodilators, diuretics, antiulcer drugs, intestinal regulators, osteoporosis drugs, antitussive and expectorant drugs, antiasthmatic drugs, antibacterial agents, frequent urination improving agents, tonics, and vitamins. These pharmaceutically active components may be used alone or in combination of two or more kinds thereof.

Specific examples of the pharmaceutically active component include pharmaceutically active components described in "Japanese Pharmacopoeia", "Japanese Pharmaceutical Codex", "USP", "NF", and "EP", such as aspirin, aspirin aluminum, acetaminophen, ethenzamide, sasapyrin, salicylamide, lactyl phenetidine, isothypendyl hydrochloride, diphenylpyraline hydrochloride, diphenhydramine hydrochloride, difeterol hydrochloride, triprolidine hydrochloride, tripelenamine hydrochloride, thonzylamine hydrochloride, fenethazine hydrochloride, methdilazine hydrochloride, diphenhydramine salicylate, carbinoxamine diphenyldisulfonate, alimemazine tartrate, diphenhydramine tannate, diphenylpyraline teoclate, mebhydroline napadisilate, promethazine methylenedisalicylate, carbinoxamine maleate, dl-chlorpheniramine maleate, d-chlorpheniramine maleate, difeterol phosphate, alloclamide hydrochloride, cloperastine hydrochloride, pentoxyverine citrate (carbetapentane citrate), tipepidine citrate, dibunate sodium, dextromethorphan hydrobromide, dextromethorphan phenolphthalic acid, tipepidine hibenzate, cloperastine fendizoate, codeine phosphate, dihydrocodeine phosphate, noscapine hydrochloride, noscapine, dl-methylephedrine hydrochloride, dl-methylephedrine saccharin salt, potassium guaiacolsulfonate, guaifenesin, sodium benzoate, caffeine, anhydrous caffeine, vitamin B1 and derivatives thereof and salts thereof, vitamin B2 and derivatives thereof and salts thereof, vitamin C and derivatives thereof and salts thereof, hesperidin and derivatives thereof and salts thereof, vitamin B6 and derivatives thereof and salts thereof, nicotinamide, calcium pantothenate, aminoacetic acid, magnesium silicate, synthetic aluminum silicate, synthetic hydrotalcite, magnesium oxide, dihydroxyaluminum aminoacetate (aluminum glycinate), an aluminum hydroxide gel (as a dry aluminum hydroxide gel), a dry aluminum hydroxide gel, an aluminum hydroxide-magnesium carbonate mixed dry gel, a co-precipitation product of aluminum hydroxide and sodium bicarbonate, a co-precipitation product of aluminum hydroxide, calcium carbonate, and magnesium carbonate, a co-precipitation product of magnesium hydroxide and aluminum potassium sulfate, magnesium carbonate, magnesium aluminometasilicate, ranitidine hydrochloride, cimetidine, famotidine, naproxen, diclofenac sodium, piroxicam, azulene, indomethacin, ketoprofen, ibuprofen, diphenidol hydrochloride, diphenylpyraline hydrochloride, diphenhydramine hydrochloride, promethazine hydrochloride, meclizine hydrochloride, dimenhydrinate, diphenhydramine tannate, fenethazine tannate, diphenylpyraline teoclate, diphenhydramine fumarate, promethazine methylenedisalicylate, scopolamine hydrobromide, oxyphencyclimine hydrochloride, dicyclomine hydrochloride, methixene hydrochloride, atropine methyl bromide, anisotropine methyl bromide, scopolamine methyl bromide, 1-hyoscyamine methyl bromide, benactidium methyl bromide, a belladonna extract, isopropamide iodide, diphenylpiperidinomethyldioxolane iodide, papaverine hydrochloride, aminobenzoic acid, cesium oxalate, ethyl piperidylacetylaminobenzoate, aminophylline, diprophylline, theophylline, sodium bicarbonate, fursultiamine, isosorbide dinitrate, ephedrine, cephalexin, ampicillin, sulfisoxazole, sucralfate, allylisopropylacetylurea, bromvalerylurea and the like, Ephedra, Nandina, Prunus jamasakura bark, Polygala root, Licorice, platycodon, Plantago seed, Plantago herb, Senega, Fritillaria, Fennel, Phellodendron bark, Coptis rhizome, Zedoary, Chamomile, Cinnamon bark, Gentian, Bezoar, Animal gall (including fel ursi), Adenophora polymorpha root, ginger, atractylodes, clove, tangerine, atractylodes, Pheretima, ginseng, carrot, valerian, moutan peel, Japanese pepper and extracts thereof and the like, insulin, vasopressin, interferon, urokinase, serratiopeptidase, and somatostatin. These pharmaceutically active components may be used alone or in combination of two or more thereof.

The poorly water-soluble active component in the specification of the present application is, for example, a pharmaceutically active component, and refers to an active component for which the amount of water required to dissolve 1 g of a solute is 30 mL or greater in the 18th revised edition of the Japanese Pharmacopoeia. In a case of poor solubility in water, the effect can be obtained by blending the poorly water-soluble active component with the molded article according to the present embodiment as the active component, regardless of the sublimation properties and the degree of surface polarity.

Examples of the poorly water-soluble and solid active component include pharmaceutically active components described in "Japanese Pharmacopoeia", "Japanese Pharmaceutical Codex", "USP", "NF", and "EP", for example, antipyretics analgesics such as acetaminophen, ibuprofen, benzoic acid, ethenzamide, caffeine, camphor, quinine, calcium gluconate, dimethylcaprol, sulfamine, theophylline, theopromine, riboflavin, mephenesin, phenobarbital, aminophylline, thioacetazone, quercetin, rutin, salicylic acid, theophylline sodium salt, pyrapital, quinine hydrochloride, irgapyrin, digitoxin, griseofulvin, and phenacetin, neurological drugs, sedatives, hypnotics, muscle relaxants, blood pressure sclerosants, and antihistamines, antibiotics such as acetylspiramycin, ampicillin, erythromycin, xatamycin, chloramphenicol, triacetyloleandomycin, nystatin, and colistin sulfate, steroid hormones such as methyltestosterone, methylandrosteronediol, progesterone, estradiol benzoate, ethinylestradiol, deoxycorticosterone acetate, cortisone acetate, hydrocortisone, hydrocortisone acetate, and prednisolone, nonsteroidal yolk hormones such as dienstrol, hexestrol, diethylstilbestrol, diethylstilbestrol dibromo chloride, and chlorotrianisene, and other fat-soluble vitamins. These active components may be used alone or in combination of two or more kinds thereof.

Examples of the poorly water-soluble and liquid active component include pharmaceutically active components described in "Japanese Pharmacopoeia", "Japanese Pharmaceutical Codex", "USP", "NF", and "EP", for example, teprenone, indomethacin farnesyl, menatetrenone, phytonadione, vitamins such as vitamin A oil, phenipentol, vitamin D, and vitamin E, higher unsaturated fatty acids such as DHA (docosahexaenoic acid), EPA (eicosapentaenoic acid), and cod liver oil, coenzyme Q, and oil-soluble flavorings such as orange oil, lemon oil, and peppermint oil. Vitamin E has various homologues and derivatives, but is not particularly limited as long as these are in a liquid state at a normal temperature. Specific examples of such homologues and derivatives of vitamin E include dl-$\alpha$-tocopherol, dl-$\alpha$-tocopherol acetate, d-$\alpha$-tocopherol, and d-$\alpha$-tocopherol acetate. These active components may be used alone or in combination of two or more kinds thereof.

Examples of the poorly water-soluble and semi-solid active component include Chinese herbal or crude drug extracts, such as earthworms, licorice, cinnamon bark, peony, moutan pea, valerian, Japanese pepper, ginger, tangerine, ephedra, nandina, Chinese laurel, onion, bellflower, plantago seed, Chinese laurel, garlic, Seneca, fritillary, fennel, Phellodendron bark, Coptis orb., Zedoary, chamomile, gentian, bezoar, animal gall, Chinese laurel, ginger, atractylodes chinensis, cinnamon, rhizome, atractylodes chinensis, ginseng, kudzu decoction, Keishito, Kososan, Shikokeshito, Koshikoto, Koseiryuto, Bakumontoto, Hange Kobokuto, and Maoto, oyster extracts, propolis, and propolis extracts. These active components may be used alone or in combination of two or more kinds thereof.

The sublimable active component is not particularly limited as long as the component has sublimation properties. The sublimable active component may be in a solid form, a liquid form, or a semi-solid form at a normal temperature.

Examples of the sublimable active component include sublimable pharmaceutically active components described in "Japanese Pharmacopoeia", "Japanese Pharmaceutical Codex", "USP", "NF", and "EP", such as benzoic acid, ethenzamide, caffeine, camphor, salicylic acid, phenacetin, and ibuprofen. These active components may be used alone or in combination of two or more kinds thereof.


[Other components]

Examples of the excipient include those classified as excipients in "Encyclopedia of Pharmaceutical Excipients" (published by Yakuji Nippo, Ltd.) such as starch acrylate, L-aspartic acid, aminoethylsulfonic acid, aminoacetic acid, candy (powder), gum arabic, gum arabic powder, alginic acid, sodium alginate, pregelatinized starch, pumice granules, inositol, ethyl cellulose, ethylene vinyl acetate copolymer, sodium chloride, olive oil, kaolin, cocoa butter, casein, fructose, pumice granules, carmellose, carmellose sodium, hydrous silicon dioxide, dry yeast, dry aluminum hydroxide gel, dry sodium sulfate, dry magnesium sulfate, agar, agar powder, xylitol, citric acid, sodium citrate, disodium citrate, glycerin, calcium glycerophosphate, sodium gluconate, L-glutamine, clay, clay 3, clay granules, croscarmellose sodium, crospovidone, magnesium aluminosilicate, calcium silicate, magnesium silicate, light silicic anhydride, light liquid paraffin, cinnamon powder, crystalline cellulose, crystalline cellulose and carmellose sodium, crystalline cellulose (granules), Genmai koji, synthetic aluminum silicate, synthetic hydrotalcite, sesame oil, wheat flour, wheat starch, wheat germ flour, rice flour, rice starch, potassium acetate, calcium acetate, cellulose acetate phthalate, safflower oil, white beeswax, zinc oxide, titanium oxide, magnesium oxide, $\beta$-cyclodextrin, dihydroxyaluminum aminoacetate, 2,6-di-butyl-4-methylphenol, dimethylpolysiloxane, tartaric acid, potassium hydrogen tartrate, calcined gypsum, sucrose fatty acid ester, magnesium alumina hydroxide, aluminum hydroxide gel, aluminum hydroxide-sodium bicarbonate coprecipitate, magnesium hydroxide, squalane, stearyl alcohol, stearic acid, calcium stearate, polyoxyl stearate, magnesium stearate, hardened soybean oil, refined gelatin, refined shellac, refined sucrose, refined sucrose spherical granules, cetostearyl alcohol, polyethylene glycol 1000 monocetyl ether, gelatin, sorbitan fatty acid ester, D-sorbitol, tricalcium phosphate, soybean oil, soybean unsaponifiable matter, soybean lecithin, skim milk powder, talc, ammonium carbonate, calcium carbonate, magnesium carbonate, neutral anhydrous sodium sulfate, low-substituted hydroxypropyl cellulose, dextran, dextrin, natural aluminum silicate, corn starch, tragacanth powder, silicon dioxide, calcium lactate, lactose, lactose granules, perfiller 101, white shellac, white petrolatum, Hakudo, white sugar, white sugar and starch spherical granules, naked barley leaf extract powder, naked malt leaf green juice dried powder, honey, paraffin, potato starch, semi-digested starch, human serum albumin, hydroxypropyl starch, hydroxypropyl cellulose, hydroxypropyl cellulose, hydroxypropyl methylcellulose phthalate, hydroxypropyl methylcellulose phthalate, phytic acid, glucose, glucose hydrate, partially pregelatinized starch, pullulan, propylene glycol, powdered reduced maltose syrup, powdered cellulose, pectin, bentonite, sodium polyacrylate, polyoxyethylene alkyl ether, polyoxyethylene hydrogenated castor oil, polyoxyethylene (105) polyoxypropylene (5) glycol, polyoxyethylene (160) polyoxypropylene (30) glycol, sodium polystyrene sulfonate, polysorbate 80, polyvinyl acetal diethylaminoacetate, polyvinylpyrrolidone, polyethylene glycol, maltitol, maltose, D-mannitol, starch syrup, isopropyl myristate, anhydrous lactose, anhydrous calcium hydrogen phosphate, anhydrous calcium phosphate granules, magnesium aluminometasilicate, methylcellulose, cottonseed flour, cottonseed oil, Japan wax, aluminum monostearate, glycerin monostearate, sorbitan monostearate, medicinal charcoal, peanut oil, aluminum sulfate, calcium sulfate, granular corn starch, liquid paraffin, dl-malic acid, calcium hydrogen phosphate, calcium hydrogen phosphate, calcium hydrogen phosphate granules, sodium hydrogen phosphate, potassium dihydrogen phosphate, calcium dihydrogen phosphate, and sodium dihydrogen phosphate. These excipients may be used alone or in combination of two or more kinds thereof.

Examples of the disintegrating agent include those classified as disintegrating agents in "Encyclopedia of Pharmaceutical Excipients" (published by Yakuji Nippo, Ltd.), for example, celluloses such as croscarmellose sodium, carmellose, carmellose calcium, carmellose sodium, and low-substituted hydroxypropyl cellulose, starches such as sodium carboxymethyl starch, hydroxypropyl starch, rice starch, wheat starch, corn starch, potato starch, and partially pregelatinized starch, and synthetic polymers such as crospovidone and crospovidone copolymers. These disintegrating agents may be used alone or in combination of two or more kinds thereof.

Examples of the binding agent include those classified as binding agents described in "Encyclopedia of Pharmaceutical Excipients" (published by Yakuji Nippo, Ltd.), for example, saccharides such as white sugar, glucose, lactose, and fructose, sugar alcohols such as mannitol, xylitol, maltitol, erythritol, and sorbitol, water-soluble polysaccharides such as gelatin, pullulan, carrageenan, locust bean gum, agar, glucomannan, xanthan gum, tamarind gum, pectin, sodium alginate, and gum arabic, celluloses such as crystalline cellulose, powdered cellulose, hydroxypropyl cellulose, and methyl cellulose, starches such as pregelatinized starch and starch paste, synthetic polymers such as polyvinylpyrrolidone, carboxyvinyl polymer, and polyvinyl alcohol, and inorganic compounds such as calcium hydrogen phosphate, calcium carbonate, synthetic hydrotalcite, and magnesium aluminosilicate. These binding agents may be used alone or in combination of two or more kinds thereof.

Examples of the fluidizing agent include those classified as fluidizing agents described in "Encyclopedia of Pharmaceutical Excipients" (published by Yakuji Nippo, Ltd.), for example, silicon compounds such as hydrous silicon dioxide and light silicic anhydride. These fluidizing agents may be used alone or in combination of two or more kinds thereof.

Examples of the lubricant include those classified as lubricants described in "Encyclopedia of Pharmaceutical Excipients" (published by Yakuji Nippo, Ltd.) such as magnesium stearate, calcium stearate, stearic acid, sucrose fatty acid ester, and talc. These lubricants may be used alone or in combination of two or more kinds thereof.

Examples of the flavoring agent include those classified as flavoring agents described in "Encyclopedia of Pharmaceutical Excipients" (published by Yakuji Nippo, Ltd.) such as glutamic acid, fumaric acid, succinic acid, citric acid, sodium citrate, tartaric acid, malic acid, ascorbic acid, sodium chloride, and 1-menthol. These flavoring agents may be used alone or in combination of two or more kinds thereof.

Examples of the fragrance include those classified as flavoring agent and fragrances described in "Encyclopedia of Pharmaceutical Excipients" (published by Yakuji Nippo, Ltd.), for example, oranges, vanilla, strawberries, yogurt, menthol, oils such as fennel oil, cinnamon oil, spruce oil. and peppermint oil, and green tea powder. These flavoring agents and fragrances may be used alone or in combination of two or more kinds thereof.

Examples of the colorant include those classified as colorants described in "Encyclopedia of Pharmaceutical Excipients" (published by Yakuji Nippo, Ltd.), for example, edible coloring agents such as Edible Red No. 3, Edible Yellow No. 5, and Edible Blue No. 1, sodium copper chlorophyllin, titanium oxide, and riboflavin. These colorants may be used alone or in combination of two or more kinds thereof.

Examples of the sweetener include those classified as sweeteners described in "Encyclopedia of Pharmaceutical Excipients" (published by Yakuji Nippo, Ltd.) such as aspartame, saccharin, dipotassium glycyrrhizinate, stevia, maltose, maltitol, starch syrup, and amacha powder. These sweeteners may be used alone or in combination of two or more kinds thereof.

(Method of producing molded article)

A method of producing a tablet containing one or more active components and the cellulose powder of the present embodiment as main components will be described below. Further, this method is an example of the method of producing the molded article according to the present embodiment, and the effects of the molded article according to the present embodiment are not limited to the following method.

Examples of the production method include a method of mixing the active component with the cellulose powder according to the present embodiment and compression-molding the mixture. In this case, other additives may be blended with the mixture as necessary in addition to the active component. The other additives are, for example, one or more selected from components consisting of an excipient, a disintegrating agent, a binding agent, a fluidizing agent, a lubricant, a flavoring agent, a fragrance, a colorant, a sweetener, and a dissolution auxiliary agent.

The order of adding the components is not particularly limited, and examples of a method of adding the components include i-1) a method of collectively mixing the active component, the cellulose powder of the present embodiment, and other additives as necessary and compression-molding the mixture and ii-1) a method of pretreating and mixing the active component with an additive such as a fluidizing agent or a lubricant, mixing the mixture with the cellulose powder of the present embodiment and other additives as necessary, and compression-molding the mixture. A lubricant may be added to the mixed powder for compression molding, which is obtained by the method i-1) or ii-1), and the mixture may be further mixed and then compression-molded.

**In** particular, in a case of using a poorly water-soluble active component, the following production method can be employed. Examples of the production method include i-2) a method of pulverizing the active component or using the active component as it is, mixing the active component with the cellulose powder of the present embodiment and, as necessary, other components, and performing compression molding and ii-2) a method of dissolving or dispersing the active component in one or more media selected from the group consisting of water, an organic solvent, and a dissolution auxiliary agent, adsorbing the active component to the cellulose powder of the present embodiment or other additives, mixing the active component with the cellulose powder of the present embodiment or other additives as necessary, distilling off the medium as necessary, and performing compression molding.

Among these, from the viewpoints of the moldability and the fluidity, particularly, it is preferable that, in the method i-2), the active component and the additive such as a fluidizing agent are pretreated and mixed, mixed with the cellulose powder of the present embodiment and, as necessary, other components, and then compression-molded.

The crystal form of the active component before compression molding may be the same as or different from the state before the preparation. Among these, from the viewpoint of stability, it is preferable that the crystal form of the active component before compression molding is the same as that before the preparation.

In a case where a poorly water-soluble active component is used, it is effective to disperse the active component in a medium in combination with a water-soluble polymer or a surfactant as a dissolution auxiliary agent. The other additives referred to here are additives other than the cellulose powder of the present embodiment, and examples thereof include additives such as an excipient, a disintegrating agent, a binding agent, a fluidizing agent, a lubricant, a flavoring agent, a fragrance, a colorant, a sweetener, and a dissolution auxiliary agent. These additives may be used alone or in combination of two or more kinds thereof.

In particular, in a case of the method ii-2), since the step of temporarily dissolving or dispersing a poorly water-soluble or water-insoluble active component is performed, the effect of improving the elution of the active component is also obtained. In particular, in a case where a liquid dispersion medium such as polyethylene glycol is used in combination as a dispersion medium for the pharmaceutically active component, the dispersion medium obtained by dispersing the original active component, even in a case where the original active component is crystal powder, is in the liquid or semi-solid form. Therefore, in a case where the compression moldability and the fluidity are not excellent as in the cellulose powder of the present embodiment, the active component cannot be made into a tablet. In addition, in a case where polyethylene glycol or the like is used as a dispersion medium of the pharmaceutically active component, it is said that the active component has a structure coated with polyethylene glycol in the blood in a case where the active component is absorbed in the body. Therefore, an effect of sustaining the drug efficacy of the active component that is easily metabolized in the liver is also expected.

The method of adding each component is not particularly limited as long as the method is usually carried out, and for example, the components may be continuously added using a small suction transport device, a pneumatic transport device, a bucket conveyor, a pressure feed type transport device, a vacuum conveyor, a vibration type quantitative feeder, a spray, a funnel, or the like, or may be collectively added.

In a case where the active component is a solution, a suspension, or an emulsified liquid, it is preferable to employ a method of spraying the active component to the cellulose powder or other additives. In this manner, the variation in the concentration of the active component in the final product can be further reduced. Examples of the spraying method include a method of spraying an active component solution or an active component dispersion liquid using a Teva spray, a pressure nozzle, a two-fluid nozzle, a four-fluid nozzle, a rotary disk, an ultrasonic nozzle, or the like and a method of dropping an active component solution or an active component dispersion liquid from a tubular nozzle. In a case of adding an active component solution or an active component dispersion liquid, layering or coating may be performed such that the active component is laminated on the surface of the cellulose particles in the cellulose powder, the active component may be carried in the cellulose powder particles, or the active component solution or the active component dispersion liquid may be used as a binding liquid to granulate a mixture of the cellulose powder particles or the porous cellulose and other additives in a matrix form. The layering and the coating may be a wet type or a dry type.

The mixing method is not particularly limited as long as the method is usually carried out, and examples thereof include a method using a container rotary mixer such as a V-type mixer, a W-type mixer, a double cone-type mixer, or a container tuck-type mixer, a method using a stirring mixer such as a high-speed stirring mixer, a universal stirring mixer, a ribbon-type mixer, a pug-type mixer, or a kneader-type mixer, and a method using a high-speed fluid mixer, a drum-type mixer, and a fluidized bed mixer. In addition, a method of using a container shaking type mixer such as a shaker may also be used.

The compression molding method for a composition is not particularly limited as long as the method is usually carried out method, and examples thereof include a method of compression-molding into a desired shape using a mortar and a pestle and a method of cutting into a desired shape after compression-molding into a sheet shape in advance. Examples of a compression molding machine include a roller-type press machine such as a static pressure press machine, a briquetting roller-type press machine, or a smooth roller-type press machine, and a single punch tablet press and a rotary tablet press.

The method of dissolving or dispersing the active component in a medium is not particularly limited as long as the method is a usually used dissolving or dispersing method, and examples thereof include a stirring and mixing method using a stirring blade of a one-directional rotary type, a multi-axis rotary type, a reciprocating type, an up-down movement type, a rotation + up-down movement type, or a pipe line type, such as a portable mixer, a three-dimensional mixer, or a side surface mixer, a jet stream type stirring and mixing method such as a line mixer, a gas blowing type stirring and mixing method, a mixing method using a high-shear homogenizer, a high-pressure homogenizer, an ultrasonic homogenizer, or the like, and a container shaking type mixing method using a shaker.

The medium used in the above-described production method is not particularly limited as long as the medium is used for a pharmaceutical product, and for example, water or an organic solvent can be used. Examples of the organic solvent include those classified as solvents described in "Encyclopedia of Pharmaceutical Excipients" (published by Yakuji Nippo, Ltd.), for example, alcohols such as methanol, ethanol, isopropyl alcohol, butyl alcohol, 2-methylbutyl alcohol, and benzyl alcohol, hydrocarbons such as pentane, hexane, heptane, and cyclohexane, and ketones such as acetone and ethyl methyl ketone. These media may be used alone or in combination of two or more kinds thereof. The dispersion may be temporarily carried out with one kind of medium, the medium may be removed, and the dispersion may be carried out with a different medium.

Examples of the water-soluble polymer as a dissolution auxiliary agent include water-soluble polymers described in "Encyclopedia of Pharmaceutical Excipients" (published by Yakuji Nippo, Ltd.) such as hydroxypropyl cellulose, hydroxypropyl methyl cellulose, polyacrylic acid, a carboxyvinyl polymer, polyethylene glycol, polyvinyl alcohol, polyvinylpyrrolidone, methyl cellulose, ethyl cellulose, gum arabic, and starch paste. These water-soluble polymers may be used alone or in combination of two or more kinds thereof.

Examples of oils and fats as a dissolution auxiliary agent include oils and fats described in "Encyclopedia of Pharmaceutical Excipients" (published by Yakuji Nippo, Ltd.) such as stearic acid monoglyceride, stearic acid triglyceride, sucrose stearate, paraffins such as liquid paraffin, carnauba wax, hydrogenated oils such as hydrogenated castor oil, castor oil, stearic acid, stearyl alcohol, and polyethylene glycol. These oils and fats may be used alone or in combination of two or more kinds thereof.

Examples of the surfactant as a dissolution auxiliary agent include those classified as surfactants described in "Encyclopedia of Pharmaceutical Excipients" (published by Yakuji Nippo, Ltd.) such as phospholipid, glycerin fatty acid ester, polyethylene glycol fatty acid ester, sorbitan fatty acid ester, polyoxyethylene hydrogenated castor oil, polyoxyethylene cetyl ether, polyoxyethylene stearyl ether, polyoxyethylene nonylphenyl ether, polyoxyethylene polyoxypropylene glycol, polyoxyethylene sorbitan monolaurate, polysorbate, sorbitan monooleate, glycerol monostearate, monooxyethylene sorbitan monopalmitate, monooxyethylene sorbitan monostearate, polyoxyethylene sorbitan monooleate, sorbitan monopalmitate, and sodium lauryl sulfate. These surfactants may be used alone or in combination of two or more kinds thereof.

The tablet in the specification of the present application refers to a molded article that contains the cellulose powder of the present embodiment, one or more active components, and other additives as necessary, and can be obtained by compression molding. A tablet composition blended with the cellulose powder of the present embodiment is particularly a composition in which practical hardness is obtained by a simple method such as a direct tableting method without performing a complicated step. As a method of producing a tablet, a dry-type granule compression method, a wet-type granule compression method, a post-tableting method, a method of producing a multi-cored tablet having a tablet subjected to compression molding in advance as an inner core, a method of producing a multi-layered tablet in which a plurality of molded articles compressed in advance are overlapped and compressed again, or the like may be used as necessary.

The cellulose powder of the present embodiment is excellent in various physical properties required as an excipient having compression moldability, fluidity, and disintegrability. Therefore, the cellulose powder is particularly effective for a tablet containing various kinds or a large amount of drugs that are difficult to obtain tablet hardness and are likely to cause tableting defects such as fracture, chipping, peeling from the inside, and cracking on the surface of the tablet, for example, tablets containing powdered extracts of over-the-counter medicines and Chinese herbal medicines, small tablets, non-circular deformed tablets that have sites where a compression pressure is difficult to apply evenly, such as constricted edges, drugs such as enzymes and proteins that are easily inactivated by pressure or friction with excipients, and coated granule-containing tablets. In addition, since the cellulose powder of the present embodiment has excellent compression moldability, a tablet having a practical abrasion degree at a relatively low compression pressure can be obtained. Therefore, since a void (water guide pipe) can be maintained in the tablet, the cellulose powder is also effective for an orally disintegrating tablet that is rapidly disintegrated in the oral cavity.

Further, in a case of a multilayer tablet or a dry coated tablet in which components having several compositions are compression-molded in one step or in other steps, the cellulose powder is also effective for suppressing peeling and cracking between layers in addition to imparting the hardness and suppression of typical tableting defects described above. Since the cellulose powder of the present embodiment has a high proportion of primary particles, the divisibility of the particles is also excellent, and in a case where the cellulose powder is used for a scored tablet or the like, the tablet is easily uniformly divided.

Further, the cellulose powder of the present embodiment has a developed porous structure, and the cellulose particles have excellent retention of fine particle-like drugs, suspension-like drugs, and solution-like components. Therefore, the tablet obtained by using the cellulose powder also has excellent retention of fine particle-like drugs, suspensions, and solution-like components. Therefore, the cellulose powder of the present embodiment is also effective for preventing and reinforcing peeling of a layering layer, a coating layer, or a sugar-coated layer in a tablet in which a suspension-like or solution-like component is used, such as a layered or coated tablet, and a sugar-coated tablet in which components such as sugar and calcium carbonate are laminated on a surface of a tablet in a suspended state.

Next, a method of using a composition containing one or more active components and the cellulose powder will be described. The composition containing the active component in a solid, liquid, or semi-solid form obtained by the method described above and the cellulose powder may be used as a solid preparation in a powdery or granular form, or may be used as a coated powder or a granular solid preparation by further coating the powdery or granular composition with a coating agent. The coated or uncoated powder or granular composition obtained here may be used by filling a capsule, or may be used as a tablet-type solid preparation by being compression-molded. Further, the composition may be used by coating a capsule or a tablet.

Examples of the coating agent in a case of carrying out the coating include those classified as coating agents described in "Encyclopedia of Pharmaceutical Excipients" (published by Yakuji Nippo, Ltd.) such as an ethyl acrylate-methyl methacrylate copolymer dispersion, acetylglycerin fatty acid ester, an aminoalkyl methacrylate copolymer, gum arabic powder, ethyl cellulose, an ethyl cellulose aqueous dispersion, octyldecyl triglyceride, olive oil, kaolin, cocoa butter, prunella vulgaris, castor wax, caramel, carnauba wax, a carboxyvinyl polymer, carboxymethylethylcellulose, sodium carboxymethyl starch, carmellose calcium, carmellose sodium, hydrated silicon dioxide, a dried aluminum hydroxide gel, dried milky white lac, a dried methacrylic acid copolymer, winter plum powder, fish scale powder, gold leaf, silver leaf, triethyl citrate, glycerin, glycerin fatty acid ester, magnesium silicate, light anhydrous silicic acid, light anhydrous silicic acid-containing hydroxypropyl cellulose, light liquid paraffin, spermaceti, crystalline cellulose, hydrogenated oil, synthetic aluminum silicate, synthetic wax, high glucose syrup, hard wax, succinated gelatin, wheat flour, wheat starch, rice starch, cellulose acetate, a vinyl acetate resin, cellulose acetate phthalate, white beeswax, titanium oxide, magnesium oxide, a dimethylaminoethyl methacrylate-methyl methacrylate copolymer, dimethylpolysiloxane, a dimethylpolysiloxane/silicon dioxide mixture, a silicon oxide mixture, calcined gypsum, sucrose fatty acid ester, zinc powder, aluminum hydroxide gel, hydrogenated rosin glycerin ester, stearyl alcohol, stearic acid, aluminum stearate, calcium stearate, polyoxyl stearate, magnesium stearate, refined gelatin, refined shellac, refined white sugar, zein, sorbitan sesquioleate, cetyl alcohol, gypsum, gelatin, shellac, sorbitan fatty acid ester, D-sorbitol, D-sorbitol liquid, tribasic calcium phosphate, talc, calcium carbonate, magnesium carbonate, simple syrup, medium gold leaf, precipitated calcium carbonate, low-substituted hydroxypropyl cellulose, a terpene resin, starch (soluble), corn syrup, corn oil, triacetin, calcium lactate, white shellac, white sugar, honey, hard fat, paraffin, pearl powder, potato starch, hydroxypropyl cellulose, hydroxypropyl cellulose, hydroxypropyl cellulose acetate succinate, a hydroxypropyl cellulose/titanium oxide/polyethylene glycol mixture, hydroxypropyl methylcellulose phthalate, piperonyl butoxide, castor oil, diethyl phthalate, dibutyl phthalate, butyl phthalate glycolate, glucose, partially pregelatinized starch, a fumaric acid/stearic acid/polyvinyl acetal diethylaminoacetate/hydroxypropyl cellulose mixture, pullulan, propylene glycol, powdered sugar, bentonite, povidone, polyoxyethylene hydrogenated castor oil, polyoxyethylene (105) polyoxypropylene (5) glycol, polyoxyethylene (160) polyoxypropylene (30) glycol, polyoxyethylene sorbitan monostearate, polyvinyl acetal diethylaminoacetate, polyvinyl alcohol (partially saponified), polyethylene glycol, a hydroxyl-terminated methylpolysiloxane silicone resin copolymer, D-mannitol, starch syrup, beeswax, myristyl alcohol, an anhydrous silicic acid hydrate, phthalic anhydride, anhydrous calcium hydrogen phosphate, a methacrylic acid copolymer, magnesium aluminometasilicate, methylcellulose, a 2-methyl-5-vinylpyridine methylacrylate-methacrylic acid copolymer, Japan wax, glycerin monostearate, sorbitan monostearate, sorbitan monolaurate, montan acid ester wax, medicinal charcoal, lauromacrogol, calcium sulfate, a liquid coumarone resin, liquid paraffin, dl-malic acid, calcium hydrogen phosphate, calcium hydrogen phosphate, sodium hydrogen phosphate, calcium dihydrogen phosphate, and rosin. These coating agents may be used alone or in combination of two or more kinds thereof.

The cellulose powder of the present embodiment has a developed porous structure, and the particles have excellent drug retention. Therefore, the particles in which the drug is carried in the pores may be used as fine particles as they are, may be used as granules by granulation, or may be compression-molded. The fine granules, granules, or tablets may be further coated.

The method of carrying a drug in the pores of the cellulose powder is not particularly limited as long as the method is a known method, and examples thereof include i) a method of mixing the cellulose powder with a fine particle-like drug and carrying the drug in the pores, ii) a method of mixing a powdery drug with the cellulose powder under high shear and forcedly carrying the drug in the pores, iii) a method of mixing a drug that has once been a solution or a dispersion liquid with the cellulose powder, carrying the drug in the pores, and drying and carrying the drug as necessary, iv) a method of mixing a sublimable drug with the cellulose powder and sublimating and adsorbing the drug in the pores by heating or depressurizing, and v) a method of mixing a drug with the cellulose powder before or during heating and carrying the melted drug in the pores. These methods may be used alone or in combination of two or more thereof.

The cellulose powder of the present embodiment has a developed pore structure and has appropriate water retention and oil retention. Therefore, the core particles can also be used for layering or coating, in addition to the applications as an excipient. **In** this case, there is an effect of suppressing aggregation between particles in the layering or coating step. The layering or coating may be a dry type or a wet type.

In addition, in a case where the active component is a solution, a suspension, or an emulsified liquid, a method of immersing the cellulose powder or a mixture of the cellulose powder and other additives used as a carrier for dipping in an active component solution, a suspension, or an emulsified liquid to maintain the active component is employed. Although the conditions such as the type and the concentration of the active component vary, even in a case of a liquid immersion method such as dipping, the uniformity of the active component is practically maintained, and the method is excellent in that the steps are simpler than those of the above-described spraying.

Further, in a case where the active component is a solution, a suspension, or an emulsified liquid, a method of using cellulose powder or a mixture of the cellulose powder and other additives as a carrier, immersing the cellulose powder or the mixture in an active component solution, a suspension, or an emulsified liquid, and then spray-drying the dispersion liquid to obtain a complex may be employed.

The cellulose powder before and after the addition of the active component solution or the active component dispersion liquid, or a mixture of the cellulose powder and other additives may be in a state in which the unit particles are individually dispersed, or may be in the form of an aggregated granulated product.

Examples of a granulation method in a case of undergoing granulation in the production step include dry-type granulation, wet-type granulation, heat granulation, spray granulation, and microencapsulation. Specifically, as the wet-type granulation method, a fluidized bed granulation method, a stirring granulation method, an extrusion granulation method, a crushing granulation method, or a rolling granulation method is effective. In the fluidized bed granulation method, a binding liquid is sprayed to fluidized powder in a fluidized bed granulation device to perform granulation. In the stirring granulation method, the stirring blade is allowed to rotate in the mixing tank while adding the binding liquid, whereby mixing, kneading, and granulation of the powder are simultaneously performed in the sealed structure. In the extrusion granulation method, the wet mass that has been kneaded by the addition of the binding liquid is granulated by forcibly extruding the wet mass from a screen having an appropriate size by a method such as a screw type method or a basket type method. In the crushing granulation method, the wet mass that has been kneaded by the addition of the binding liquid is sheared or crushed with a rotary blade of a granulator, and is granulated by being thrown out from a screen on the outer periphery by centrifugal force. In the rolling granulation method, the rolling is performed by a centrifugal force of a rotating rotor, and spherical granules having a uniform particle diameter are grown in a snowball manner by a binding liquid sprayed from a spray gun during the rolling, to perform granulation.

Examples of a method of drying the granulated product include a hot air heating type (shelf drying, vacuum drying, fluidized bed drying), a conduction heat transfer type (a flat pan type, a shelf box type, or a drum type), and freeze drying. In the hot air heating type, hot air is brought into direct contact with a material, and at the same time, evaporated moisture is removed. In the conduction heat transfer type, a material is indirectly heated through a heat transfer wall. In the freeze drying, a material is frozen at -10°C or higher and -40°C or lower, and then heated in a high vacuum ($1.3 \times 10^{-5}$ MPa or greater and $2.6 \times 10^{-4}$ MPa or less) to sublimate and remove water.

As described above, examples of the method of producing a tablet include i) a method in which a mixture of an active component and cellulose powder or a mixture of one or more kinds of active components, cellulose powder, and other additives as necessary is compression-molded by a typical method (direct tableting method), ii) a method in which an active component, cellulose powder, and other additives as necessary are mixed, granulated to obtain granules, and compression-molded by a typical method (wet-type or dry-type granule compression method), and iii) a method in which an active component, cellulose powder, and other additives as necessary are mixed and granulated to obtain granules, and cellulose powder and other additives as necessary are further mixed and compression-molded by a typical method (wet-type or dry-type granule post-compression method).

The method of adding one or more active components, cellulose powder, other additives, or granules is not particularly limited as long as the method is a usually used method, and for example, these may be continuously added using a small suction transport device, a pneumatic transport device, a bucket conveyor, a pressure feed type transport device, a vacuum conveyor, a vibration type quantitative feeder, a spray, a funnel, or the like, or may be collectively added.

In addition to being used as a tablet by being compression-molded as described above, a composition containing the active component and the cellulose powder is also excellent in the retention of solid or liquid components, and thus may be used as a granulating agent or a scattering agent, particularly for the purpose of improving fluidity, blocking resistance, and aggregation resistance. Examples of a method of producing a granulating agent or a scattering agent include dry-type granulation, wet-type granulation, heat granulation, spray drying, and microencapsulation.

[Physical properties of tablet]

In a case where the molded article according to the present embodiment is a tablet having a mass of about 370 mg which is obtained by putting (1) 603.2 parts by mass of ascorbic acid (manufactured by Watanabe Chemical Co., Ltd., used after sieving through a sieve having a sieve opening of 2 mm, and the obtained ascorbic acid having an average particle diameter $D_{50}$ of 320 $\mu$m), 108.8 parts by mass of spray-dried lactose (SuperTab 11SD (trade name), manufactured by DFE Pharma), and 80 parts by mass of the cellulose powder of the present embodiment into a polybag, shaking and mixing the mixture for 3 minutes, adding 8 parts by mass of calcium stearate (manufactured by Taihei Chemical Industry Co., Ltd.), further mixing the mixture for 30 seconds to obtain formulated powder (final composition in terms of mass ratio, ascorbic acid/spray-dried lactose/cellulose powder/calcium stearate = 75.4/13.6/10/1), supplying the obtained formulated powder by an open feeder, and performing tableting at a turntable rotation speed of 54 rpm using a rotary tablet press (manufactured by Kikusui Seisakusho Ltd., 12-tablet capacity) and a die having a diameter of 9 mm and R, a tablet having a mass of about 410 mg which is obtained by putting (2) 544.4 parts by mass of ascorbic acid (manufactured by Watanabe Chemical Co., Ltd., used after sieving through a sieve having a sieve opening of 2 mm, and the obtained ascorbic acid having an average particle diameter $D_{50}$ of 320 $\mu$m), 167.6 parts by mass of spray-dried lactose (SuperTab 11SD (trade name), manufactured by DFE Pharma), and 80 parts by mass of the cellulose powder of the present embodiment into a polybag, shaking and mixing the mixture for 3 minutes, adding 8 parts by mass of calcium stearate (manufactured by Taihei Chemical Industry Co., Ltd.), further mixing the mixture for 30 seconds to obtain formulated powder (final composition in terms of mass ratio, ascorbic acid/spray-dried lactose/cellulose powder/calcium stearate = 68/21/10/1), supplying the obtained formulated powder by an open feeder, and performing tableting at a turntable rotation speed of 54 rpm using a rotary tablet press (manufactured by Kikusui Seisakusho Ltd., 12-tablet capacity) and a die having a diameter of 9 mm and R, or a tablet having a mass of about 470 mg which is obtained by putting (3) 200 parts by mass of ethenzamide (manufactured by Yamamoto Corporation Co., Ltd., C grade), 245 parts by mass of spray-dried lactose (SuperTab 11SD (trade name), manufactured by DFE Pharma), 50 parts by mass of the cellulose powder of the present embodiment, and 5 parts by mass of light anhydrous silicic acid (AEROSIL 200 (trade name), manufactured by Nippon Aerosil Co., Ltd.) into a polybag, shaking and mixing the mixture for 3 minutes, adding 5 parts by mass of magnesium stearate (manufactured by Taihei Chemical Industry Co., Ltd.), further mixing the mixture for 30 seconds to obtain formulated powder (final composition in terms of mass ratio, ascorbic acid/spray-dried lactose/cellulose powder/calcium stearate = 40/49/10/1/1), and performing tableting on the obtained formulated powder at a speed at which 30 tablets are obtained for 1 minute using a single-shot type tablet press (manufactured by Ichikawa Precision Machine Co., Ltd.) and a die having a diameter of 8 mm and R, the variation in tablet weight is preferably 1.0% by mass or less, more preferably 0.7% by mass or less, still more preferably 0.6% by mass or less, and particularly preferably 0.5% by mass or less. Meanwhile, it is preferable that the variation in tablet mass is as small as possible, and the lower limit thereof is not particularly limited, but is usually about 0.01% by mass.

The variation in tablet mass can be measured, for example, by a method described in examples described below.

In addition, in a case where the molded article according to the present embodiment is a tablet produced under the above-described conditions, the hardness thereof is preferably 40 N or greater, more preferably 45 N or greater, still more preferably 50 N or greater, and particularly preferably 55 N or greater. Meanwhile, it is preferable that the tablet hardness is as large as possible, and the upper limit thereof is not particularly limited, but is usually about 150 N.

The tablet hardness can be measured, for example, by a method described in examples described below.

In addition, in a case where the molded article according to the present embodiment is a tablet produced under the above-described conditions, the disintegration time is preferably 1,800 seconds or shorter, more preferably 1,500 seconds or shorter, and still more preferably 1,200 seconds or shorter. Meanwhile, it is preferable that the disintegration time is as short as possible, and the lower limit thereof is not particularly limited, but is usually about 10 seconds.

The disintegration time can be measured, for example, by a method described in examples described below.

In addition, in a case where the molded article according to the present embodiment is a tablet produced under the above-described conditions, the abrasion degree thereof is preferably 1.0% by mass or less, more preferably 0.5% by mass or less, still more preferably 0.4% by mass or less, and particularly preferably 0.3% by mass or less. Meanwhile, it is preferable that the abrasion degree is as small as possible, and the lower limit thereof is not particularly limited, but is usually about 0.01% by mass.

The abrasion degree can be measured, for example, by a method described in examples described below.

That is, in a case where the molded article according to the present embodiment is a tablet produced under the above-described conditions, it is preferable that the tablet has a variation of 1.0% by mass or less in tablet mass, a tablet hardness of 40 N or greater, a disintegration time of 1,800 seconds or shorter, and an abrasion degree of less than 0.5% by mass.

Examples

An explanation will be given of the present invention based on examples. However, the embodiments of the present invention are not limited to the description of these examples.

The measuring methods and evaluation methods for each physical property in the examples and the comparative examples are as follows.

<Method of measuring physical properties>

[Physical property 1]

(Average particle diameter of cellulose powder)

The average particle diameter $D_{50}$ of the cellulose powder was determined using a laser diffraction/scattering-type particle size distribution analyzer (LA-950 V2 type (trade name), manufactured by Horiba, Ltd.). About 1.0 g of cellulose powder as a sample was placed on a sample shooter for powder, the sample was dispersed under conditions of a feeder strength of 160 and a compressed air pressure of 0.03 MPa, the scattered light measurement was performed in a range of a laser light (red) transmittance of 95% or greater and 98% or less, and a particle diameter of a cumulative volume of 50% measured was defined as an average particle diameter $D_{50}$. The measurement was performed twice, and the average value thereof was used as the average particle diameter.

[Physical property 2]

(Average degree of polymerization of cellulose powder)

About 1.3 g of cellulose powder (about 0.25 g in a case of powdered cellulose) was precisely weighed as a sample and placed in a 125 mL Erlenmeyer flask, and 25 mL of water and 25 mL of a 1 mol/L copper ethylenediamine test solution were each accurately added thereto. The mixture was immediately allowed to pass through nitrogen, tightly sealed, and dissolved while being shaken using a shaker. The appropriate amount of the liquid was accurately weighed, and a test was performed at $25 \pm 0.1°C$ using a capillary viscometer in which an approximate constant (K) of the viscometer was 0.03 to determine a kinematic viscosity v by the first method <2.53> of the viscosity measuring method. Separately, 25 mL of water and 25 mL of a 1 mol/L copper ethylenediamine test solution were accurately weighed, and a test is performed on the mixed solution using a capillary viscometer in which an approximate constant (K) of the viscometer was 0.01 to determine a kinematic viscosity v0 by the same method. A relative viscosity $\eta_{rel}$ of the cellulose powder was determined by the following equation.

$$\eta_{rel} = v/v0$$

As listed in Tables 1 and 2, a product $[\eta]C$ of a limiting viscosity $[\eta]$ (mL/g) and a concentration C (g/100 mL) was obtained from the relative viscosity $\eta_{rel}$, and an average degree P of polymerization was calculated by the following equation. Further, in the following equation, MT represents a weighed amount (g) of the cellulose powder in terms of a dry substance.

$$P = 95 [\eta]C/MT$$

[Physical property 3]

(Water absorption rate of cellulose powder)

The water absorption rate was determined using a commercially available water absorption rate measuring device (PENETO ANALYZER PNT-N type (trade name), manufactured by Hosokawa Micron Corporation). Specifically, a Teflon (registered trademark) container was filled with about 5.0 g of cellulose powder that had passed through a sieve having an opening size of 500 $\mu$m, and tapping was performed under the conditions that the number of times of performing tapping was 300 times, the stroke length was 18 mm, and the mass of the weight was 198 g. The powder after tapping was sufficiently immersed in 300 mL of pure water at a rate of 0.5 mm/s until the cellulose powder entered in a saturated state, and the square ($g^2$) of the permeation rate of pure water until the cellulose powder entered the saturated state during the measurement time was measured by the above-described measuring device. A graph of the square ($g^2$) of the permeation rate until the cellulose powder entered the saturated state and the measurement time was linearized by the least squares method, and the slope of the linearized graph was defined as the water absorption rate ($g^2$/s). The measurement was performed 5 times, and the average value thereof was used as the water absorption rate.

[Physical property 4]

(Cohesive force F of cellulose powder)

The cohesive force of the cellulose powder was calculated using a powder layer shearing force measuring device (NS-S300 type (trade name), manufactured by Nano Seeds Corporation). Specifically, a shear cell ($\varphi$15 mm, upper and lower cell lengths: 34 mm, 5 mm) was filled with powder of the cellulose powder, and the upper surface of the powder layer was flattened. Thereafter, a shearing test was performed at a shearing rate of 50 $\mu$m/sec with a target indentation load of 20 N as a condition for indentation control. In a case where the load reached the target load, the indentation was stopped, the lateral sliding was started, the static friction coefficient and the dynamic friction coefficient were measured. In the analysis, the elapsed time (s) of the measurement was plotted on the horizontal axis and the load (N) applied to the shear surface was plotted on the vertical axis, the maximum value and the minimum value of the shear surface load were determined, and the cohesive force defined by the following equation was calculated. The measurement was performed twice, and the average value thereof was used as the cohesive force.

[Cohesive force F (N%)] = ([maximum value of shear surface load (N)] - [minimum value of shear surface load (N)])/ [maximum value of shear surface load (N)] $\times$ 100

[Physical property 5]

(Powder dynamic friction angle of cellulose powder)

The powder dynamic friction angle of the cellulose powder was calculated using a powder layer shearing force measuring device (NS-S300 type (trade name), manufactured by Nano Seeds Corporation). Specifically, a shear cell ($\varphi$15 mm, upper and lower cell lengths: 34 mm, 5 mm) was filled with powder of the cellulose powder, and the upper surface of the powder layer was flattened. Thereafter, a shearing test was performed at a shearing rate of 50 $\mu$m/sec with a target indentation load of 20 N, 30 N, and 40 N as a condition for indentation control using the above-described measuring device. In a case where the load reached the target load, the indentation was stopped, the lateral sliding was started, and the static friction coefficient and the dynamic friction coefficient were measured. **In** the analysis, first, the elapsed time (s) of the measurement was plotted on the horizontal axis and the shearing force (N) was plotted on the vertical axis, and the maximum value of the shearing force and the indentation load value in this case were determined. The result was plotted on a graph in which the vertical axis was the shear stress and the horizontal axis was the normal stress, and the angle of the straight line connecting the origin and these points with respect to the X-axis was calculated as the powder dynamic friction angle. The measurement was performed twice, and the average value thereof was used as the powder dynamic friction angle.

[Physical property 6]

(Loose bulk density of cellulose powder)

The loose bulk density of the cellulose powder was measured by the method described below.
A 25 mL cylindrical metal container was filled with the cellulose powder using a Scott volumeter (model ASTM B-329-85, manufactured by Tsutsui Rikagaku Kikai Co., Ltd.). The cellulose powder contained in the 25 mL cylindrical metal container was scraped off, and the loose bulk density ($g$/cm$^3$) was calculated by dividing the mass ($g$) of the cellulose powder contained in the container by 25 mL. The measurement was performed twice, and the average value thereof was used as the loose bulk density.

[Physical property 7]

(Compression ratio of cellulose powder)

The compression ratio of the cellulose powder was measured by the method described below.

The packed bulk density was measured with a powder property evaluation device (powder tester, manufactured by HOSOKAWA MICRON CORPORATION). In this case, a sieve having an opening size of 710 $\mu$m and a metal funnel having an inner diameter of 0.8 cm was used, and the VIBRATION was set to 2.0 (supply source: AC 100 V, 60 Hz). The loose bulk density was measured by the method described above, and the compression ratio defined by the following equation was calculated. The measurement was performed twice, and the average value thereof was used as the compression ratio.

[Compression ratio (%)] = ([Packed bulk density (g/cm$^3$)] - [loose bulk density (g/cm$^3$])/[packed bulk density (g/cm$^3$)] $\times$ 100

[Physical property 8]

(Primary particle ratio of cellulose powder)

The primary particle ratio of the cellulose powder was calculated using a dry image analysis device (Malvern Morphorogi G3S) and image processing software. Specifically, first, 10,000 particles were imaged and subjected to image analysis to determine the particle diameter and the aspect ratio of each particle equivalent to the area of the circle. As a result, particle images in which the particle diameter equivalent to the area of the circle was in a range of 30 $\mu$m to 90 $\mu$m and the aspect ratio was the average value of the aspect ratios of 10,000 particles + 0.1 or less were extracted. Particles in which the brightness dispersion value of each of the extracted particle images was selected from a range of 1 to 26 were determined as primary particles, and particles in which the brightness dispersion value thereof was selected from a range of 26 to 100 were determined as secondary particles, and the primary particle ratio (%) was calculated by "number of primary particles/total number of particles $\times$ 100".

[Physical property 9]

(L/D of primary particles of cellulose powder)

Particle images of the primary particles were extracted by the same method as described above using a dry image analysis device (Malvern Morphorogi G3S) and image processing software. The ratio of major axis (L)/minor axis (D) was calculated from the major axis (L) and the minor axis (D) obtained from each of the extracted particle images, and the average value of the ratios of all the extracted particle images of the primary particles was calculated as the ratio L/D of the primary particles of the cellulose powder.

<Evaluation method>

[Production of 370 mg tablet]

603.2 g of ascorbic acid (manufactured by Watanabe Chemical Co., Ltd., used after sieving through a sieve having a sieve opening of 2 mm, and the obtained ascorbic acid having an average particle diameter of 320 $\mu$m), 108.8 g of spray-dried lactose (SuperTab 11SD (trade name), manufactured by DFE Pharma), and 80 g of each cellulose powder were put into a polybag, and shaken for 3 minutes to be mixed. Next, 8 g of calcium stearate (manufactured by Taihei Chemical Industry Co., Ltd.) was added to the mixed powder, and the mixture was further mixed for 30 seconds to obtain final formulated powder. The final composition of the final formulated powder was ascorbic acid/spray-dried lactose/each cellulose powder/calcium stearate = 75.4/13.6/10/1 in terms of mass ratio.

Next, the final formulated powder was tableted using a rotary tablet press (manufactured by Kikusui Seisakusho Ltd.. 12-tablet capacity) to obtain a tablet having a mass of about 370 mg. The formulation powder was supplied by an open feeder and tableted at a turntable rotation speed of 54 rpm using a die having a diameter of 9 mm and R. The tableting pressure was appropriately adjusted so that the tablet hardness was 40 N or greater.

[Production of 410 mg tablet]

544.4 g of ascorbic acid (manufactured by Watanabe Chemical Co., Ltd., used after sieving through a sieve having a sieve opening of 2 mm, and the obtained ascorbic acid having an average particle diameter of 320 μm), 167.6 g of spray-dried lactose (SuperTab 11SD (trade name), manufactured by DFE Pharma), and 80 g of each cellulose powder were put into a polybag and shaken for 3 minutes to be mixed. Next, 8 g of calcium stearate (manufactured by Taihei Chemical Industry Co., Ltd.) was added to the mixed powder, and the mixture was further mixed for 30 seconds to obtain final formulated powder. The final composition of the final formulated powder was ascorbic acid/spray-dried lactose/each cellulose powder/calcium stearate = 68/21/10/1 in terms of mass ratio.

Next, the final formulated powder was tableted using a rotary tablet press (manufactured by Kikusui Seisakusho Ltd., 12-tablet capacity) to obtain a tablet having a mass of about 410 mg. The formulation powder was supplied by an open feeder and tableted at a turntable rotation speed of 54 rpm using a die having a diameter of 9 mm and R. The tableting pressure was appropriately adjusted so that the tablet hardness was 40 N or greater.

[Production of 470 mg tablet]

200 g of ethenzamide (manufactured by Yamamoto Corporation Co., Ltd., C grade), 245 g of spray-dried lactose (SuperTab 11 SD (trade name), manufactured by DFE Pharma), 50 g of each cellulose powder, and 5 g of light anhydrous silicic acid (AEROSIL 200 (trade name), manufactured by Nippon Aerosil Co., Ltd.) were put into a polybag and shaken for 3 minutes to be mixed. Next, 5 g of magnesium stearate (manufactured by Taihei Chemical Industry Co., Ltd.) was added to the mixed powder, and the mixture was further mixed for 30 seconds to obtain final formulated powder. The final composition of the final formulated powder was ethenzamide/spray-dried lactose/each cellulose powder/light anhydrous silicic acid/magnesium stearate = 40/49/10/1/1 in terms of mass ratio.

Next, the final formulated powder was tableted using a single-shot type tablet press (manufactured by Ichikawa Precision Machine Co., Ltd.) to obtain a tablet having a mass of about 470 mg. The formulation powder was tableted at a speed at which 30 tablets were obtained for 1 minute using a die having a diameter of 8 mm and R. The tableting pressure was appropriately adjusted so that the tablet hardness was 100 N or greater.

[Evaluation 1]

(Variation in tablet mass (% by mass))

The masses of the obtained 10 tablets were measured, the average mass and the standard deviation (g) of the mass were calculated, and the variation in mass was evaluated from the coefficient of variation (% by mass; hereinafter, the coefficient of variation is also referred to as mass CV) defined by the following equation. It can be evaluated that the variation decreases as the coefficient of variation decreases, and it can be evaluated that the variation is satisfactory in a case where the mass CV is 1.0% by mass or less.

$$[\text{Mass CV (\% by mass)}] = [\text{standard deviation (g)}]/[\text{average mass (g)}] \times 100$$

[Evaluation 2]

(Tablet hardness (N))

The obtained tablet was destroyed by applying a load in the diameter direction of the tablet using a Schleuniger hardness tester (6D type (trade name), manufactured by Freund Corporation), and the load (N) during destruction was measured. The average value of 10 tablets was used as the tablet hardness.

[Evaluation 3]

(Disintegration time (seconds))

A disintegration test was carried in conformity with the 18th revised edition of Japanese Pharmacopoeia, a typical test method, and a tablet disintegration test method. The disintegration time of the tablet in pure water at 37°C was determined using a disintegration tester (NT-40HS type (trade name), manufactured by Toyama Sangyo Co., Ltd., no disk). The average value of 6 tablets was used as the disintegration time. It was evaluated that tables in which the disintegration time was 1,800 seconds or shorter had excellent disintegrability.

[Evaluation 4]

(Abrasion degree (%))

The weights (Wa) (g) of 18 tablets were measured, the tablets were placed in a tablet abrasion degree tester (PT-F30 ERA, manufactured by PHARMA TEST), the tablets were allowed to rotate at 25 rpm for 4 minutes, fine powder attached to the tablets was removed, the weights (Wb) (g) thereof were measured again, and the abrasion degree was calculated by the following equation.

$$[\text{Abrasion degree } (\%)] = 100 \times (\text{Wa} - \text{Wb})/\text{Wa}$$

[Example 1]

(Production of cellulose powder A)

3 kg of commercially available pulp (average degree of polymerization: 1,667) that had been shredded and 30 L of a 0.045 mol/L hydrochloric acid aqueous solution were placed in a low-speed type stirrer (manufactured by Kobelco Eco-Solutions Co., Ltd, 50 LGL reactor), hydrolyzed at 117°C for 75 minutes while being stirred at a stirring speed of 62 rpm, and neutralized with aqueous ammonia, thereby obtaining an acid-insoluble residue. The obtained acid-insoluble residue was filtered using a Nutsche so that the solid content concentration reached 34% by mass, thereby obtaining a filtration residue. Thereafter, the mixture was put into a stainless steel tank having a volume of 20 L, pure water was added thereto, and the mixture was stirred with a stirrer (model: KP-4003, HANWA AGITATOR, manufactured by Hanwa Engineering Co., Ltd., stirring blade diameter: about 17 cm) at a stirring speed of 75 rpm to obtain a cellulose dispersion liquid having a solid content concentration of 6.3% by mass. The average particle diameter of the cellulose particles in the cellulose dispersion liquid was 25 $\mu$m. This cellulose dispersion liquid was spray-dried (dispersion liquid supply rate: 18 kg/hour, inlet temperature: 180°C or higher and 220°C or lower, outlet temperature: 90°C or higher and 110°C or lower), thereby obtaining cellulose powder A.

[Example 2]

(Production of cellulose powder B)

3 kg of commercially available pulp (average degree of polymerization: 1,667) that had been shredded and 30 L of a 0.045 mol/L hydrochloric acid aqueous solution were placed in a low-speed type stirrer (manufactured by Kobelco Eco-Solutions Co., Ltd, 50 LGL reactor), hydrolyzed at 123°C for 75 minutes while being stirred at a stirring speed of 80 rpm, and neutralized with aqueous ammonia, thereby obtaining an acid-insoluble residue. The obtained acid-insoluble residue was filtered using a Nutsche so that the solid content concentration reached 37% by mass, thereby obtaining a filtration residue. Thereafter, the mixture was put into a stainless steel tank having a volume of 20 L, pure water was added thereto, and the mixture was stirred with a stirrer (HANWA AGITATOR, manufactured by Hanwa Engineering Co., Ltd., stirring blade diameter: about 17 cm) at a stirring speed of 75 rpm to obtain a cellulose dispersion liquid having a solid content concentration of 6.4% by mass. The average particle diameter of the cellulose particles in the cellulose dispersion liquid was 23 $\mu$m. This cellulose dispersion liquid was spray-dried (dispersion liquid supply rate: 22 kg/hour, inlet temperature: 180°C or higher and 220°C or lower, outlet temperature: 90°C or higher and 110°C or lower), thereby obtaining cellulose powder B.

[Example 3]

(Production of cellulose powder C)

3 kg of commercially available pulp (average degree of polymerization: 1,667) that had been shredded and 30 L of a 0.045 mol/L hydrochloric acid aqueous solution were placed in a low-speed type stirrer (manufactured by Kobelco Eco-Solutions Co., Ltd, 50 LGL reactor), hydrolyzed at 113°C for 75 minutes while being stirred at a stirring speed of 90 rpm, and neutralized with aqueous ammonia, thereby obtaining an acid-insoluble residue. The obtained acid-insoluble residue was filtered using a Nutsche, thereby obtaining a filtration residue. Thereafter, the mixture was put into a stainless steel tank having a volume of 20 L, pure water was added thereto, and the mixture was stirred with a stirrer (HANWA AGITATOR, manufactured by Hanwa Engineering Co., Ltd., stirring blade diameter: about 17 cm) at a stirring speed of 75 rpm to obtain a cellulose dispersion liquid having a solid content concentration of 6.2% by mass. The average particle diameter of the cellulose particles in the cellulose dispersion liquid was 27 µm. This cellulose dispersion liquid was spray-dried (dispersion liquid supply rate: 21 kg/hour, inlet temperature: 180°C or higher and 220°C or lower, outlet temperature: 90°C or higher and 110°C or lower), thereby obtaining cellulose powder C.

[Example 4]

(Production of cellulose powder D)

2.3 kg of commercially available pulp (average degree of polymerization: 1,667) that had been shredded and 35 L of a 0.045 mol/L hydrochloric acid aqueous solution were placed in a low-speed type stirrer (manufactured by Kobelco Eco-Solutions Co., Ltd, 50 LGL reactor) and hydrolyzed at 117°C for 75 minutes while being stirred at a stirring speed of 240 rpm, thereby obtaining an acid-insoluble residue. The obtained acid-insoluble residue was filtered using a Nutsche so that the solid content concentration reached 43% by mass, thereby obtaining a filtration residue. Thereafter, the mixture was put into a stainless steel tank having a volume of 20 L, pure water was added thereto, and the mixture was stirred with a stirrer (HANWA AGITATOR, manufactured by Hanwa Engineering Co., Ltd., stirring blade diameter: about 17 cm) at a stirring speed of 75 rpm to obtain a cellulose dispersion liquid having a solid content concentration of 5.8% by mass. Further, the cellulose dispersion liquid was further neutralized with aqueous ammonia. The average particle diameter of the cellulose particles in the cellulose dispersion liquid was 27 µm. This cellulose dispersion liquid was spray-dried (dispersion liquid supply rate: 22 kg/hour, inlet temperature: 180°C or higher and 220°C or lower, outlet temperature: 90°C or higher and 110°C or lower), thereby obtaining cellulose powder D.

[Example 5]

(Production of cellulose powder E)

2.3 kg of commercially available pulp (average degree of polymerization: 1,667) that had been shredded and 35 L of a 0.045 mol/L hydrochloric acid aqueous solution were placed in a low-speed type stirrer (manufactured by Kobelco Eco-Solutions Co., Ltd, 50 LGL reactor) and hydrolyzed at 117°C for 70 minutes while being stirred at a stirring speed of 240 rpm thereby obtaining an acid-insoluble residue. The obtained acid-insoluble residue was filtered using a Nutsche, thereby obtaining a filtration residue. Thereafter, the mixture was put into a stainless steel tank having a volume of 20 L, pure water was added thereto, and the mixture was stirred with a stirrer (HANWA AGITATOR, manufactured by Hanwa Engineering Co., Ltd., stirring blade diameter: about 17 cm) at a stirring speed of 75 rpm to obtain a cellulose dispersion liquid having a solid content concentration of 5.0% by mass. Further, the cellulose dispersion liquid was further neutralized with aqueous ammonia. The average particle diameter of the cellulose particles in the cellulose dispersion liquid was 27 µm. This cellulose dispersion liquid was spray-dried (dispersion liquid supply rate: 22 kg/hour, inlet temperature: 180°C or higher and 220°C or lower, outlet temperature: 90°C or higher and 110°C or lower), thereby obtaining cellulose powder E.

[Example 6]

(Production of cellulose powder F)

Cellulose powder F was obtained by performing the same operations as in Example 5 except that the solid content concentration of the cellulose dispersion liquid was set to 4.5% by mass.

[Example 7]

(Production of cellulose powder G)

3 kg of commercially available pulp (average degree of polymerization: 1,667) that had been shredded and 30 L of a 0.045 mol/L hydrochloric acid aqueous solution were placed in a low-speed type stirrer (manufactured by Kobelco Eco-Solutions Co., Ltd, 50 LGL reactor), hydrolyzed at 107°C for 65 minutes while being stirred at a stirring speed of 80 rpm, and neutralized with aqueous ammonia, thereby obtaining an acid-insoluble residue. The obtained acid-insoluble residue was filtered using a Nutsche, thereby obtaining a filtration residue. Thereafter, the mixture was put into a stainless steel tank having a volume of 20 L, pure water was added thereto, and the mixture was stirred with a stirrer (HANWA AGITATOR, manufactured by Hanwa Engineering Co., Ltd., stirring blade diameter: about 17 cm) at a stirring speed of 75 rpm to obtain a cellulose dispersion liquid having a solid content concentration of 6.0% by mass. This cellulose dispersion liquid was spray-dried (dispersion liquid supply rate: 21 kg/hour, inlet temperature: 180°C or higher and 220°C or lower, outlet temperature: 90°C or higher and 110°C or lower), thereby obtaining cellulose powder G.

[Comparative Example 1]

(Production of cellulose powder H)

3 kg of commercially available pulp (average degree of polymerization: 1,296) that had been shredded and 30 L of a 0.045 mol/L hydrochloric acid aqueous solution were placed in a low-speed type stirrer (manufactured by NGK Chemitech., Ltd., 30 LGL reactor), hydrolyzed at 110°C for 60 minutes while being stirred at a stirring speed of 210 rpm, and neutralized in aqueous ammonia, thereby obtaining an acid-insoluble residue having a concentration of 2.9% by mass. The average particle diameter of the cellulose particles in the acid-insoluble residue was 30 $\mu$m. This cellulose dispersion liquid was spray-dried (dispersion liquid supply rate: 20 kg/hour, inlet temperature: 180°C or higher and 220°C or lower, outlet temperature: 90°C or higher and 110°C or lower), thereby obtaining cellulose powder H.

[Comparative Example 2]

(Production of cellulose powder I)

3 kg of commercially available pulp (average degree of polymerization: 1,296) that had been shredded and 30 L of a 0.045 mol/L hydrochloric acid aqueous solution were placed in a low-speed type stirrer (manufactured by NGK Chemitech., Ltd, 30 LGL reactor), and hydrolyzed at 120°C for 60 minutes while being stirred at a stirring speed of 210 rpm, thereby obtaining an acid-insoluble residue. The obtained acid-insoluble residue was filtered using a Nutsche so that the solid content concentration reached 33% by mass, thereby obtaining a filtration residue. Thereafter, the mixture was put into a stainless steel tank having a volume of 20 L, pure water was added thereto, and the mixture was stirred with a stirrer (HANWA AGITATOR, manufactured by Hanwa Engineering Co., Ltd., stirring blade diameter: about 10 cm) at a stirring speed of 450 rpm to obtain a cellulose dispersion liquid having a solid content concentration of 9.4% by mass. Further, the cellulose dispersion liquid was further neutralized with aqueous ammonia. The average particle diameter of the cellulose particles in the cellulose dispersion liquid was 26 $\mu$m. This cellulose dispersion liquid was spray-dried (dispersion liquid supply rate: 20 kg/hour, inlet temperature: 180°C or higher and 220°C or lower, outlet temperature: 90°C or higher and 110°C or lower), thereby obtaining cellulose powder I.

[Comparative Example 3]

(Production of cellulose powder J)

3 kg of commercially available pulp (average degree of polymerization: 1,667) that had been shredded and 30 L of a 0.045 mol/L hydrochloric acid aqueous solution were placed in a low-speed type stirrer (manufactured by Kobelco Eco-Solutions Co., Ltd, 50 LGL reactor), hydrolyzed at 123°C for 75 minutes while being stirred at a stirring speed of 90 rpm, and neutralized with aqueous ammonia, thereby obtaining an acid-insoluble residue. The obtained acid-insoluble residue was filtered using a Nutsche so that the solid content concentration reached 42% by mass, thereby obtaining a filtration residue. Thereafter, the mixture was put into a stainless steel tank having a volume of 20 L, pure water was added thereto, and the mixture was stirred with a stirrer (HANWA AGITATOR, manufactured by Hanwa Engineering Co., Ltd., stirring blade diameter: about 17 cm) at a stirring speed of 75 rpm to obtain a cellulose dispersion liquid having a solid content concentration of 6.5% by mass. The average particle diameter of the cellulose particles in the cellulose dispersion liquid was 22 $\mu$m. This cellulose dispersion liquid was spray-dried (dispersion liquid supply rate: 24 kg/hour, inlet temperature: 180°C or higher and 220°C or lower, outlet temperature: 90°C or higher and 110°C or lower), thereby obtaining cellulose powder J.

[Comparative Example 4]

(Production of cellulose powder K)

2.3 kg of commercially available pulp (average degree of polymerization: 1,667) that had been shredded and 35 L of a 0.015 mol/L hydrochloric acid aqueous solution were placed in a low-speed type stirrer (manufactured by Kobelco Eco-Solutions Co., Ltd, 50 LGL reactor), and hydrolyzed at 145°C for 70 minutes while being stirred at a stirring speed of 234 rpm, thereby obtaining an acid-insoluble residue. The obtained acid-insoluble residue was filtered using a Nutsche, the filtration residue was further washed 4 times with 70 L of pure water, neutralized with aqueous ammonia, and placed in a polybucket having a volume of 45 L, pure water was added thereto, and the mixture was stirred (stirring speed: 300 rpm) with a three-one motor (type BL1200, manufactured by HEIDON, three turbine blade, blade diameter: about 8 cm), thereby obtaining a cellulose dispersion liquid having a concentration of 16%. This cellulose dispersion liquid was spray-dried (dispersion liquid supply rate: 20 kg/hour, inlet temperature: 180°C or higher and 220°C or lower, outlet temperature: 90°C or higher and 110°C or lower), thereby obtaining cellulose powder K.

[Comparative Example 5]

(Production of cellulose powder L)

2.3 kg of commercially available pulp (average degree of polymerization: 1,667) that had been shredded and 35 L of a 0.024 mol/L hydrochloric acid aqueous solution were placed in a low-speed type stirrer (manufactured by Kobelco Eco-Solutions Co., Ltd, 50 LGL reactor), and hydrolyzed at 140°C for 110 minutes while being stirred at a stirring speed of 234 rpm, thereby obtaining an acid-insoluble residue. The obtained acid-insoluble residue was filtered using a Nutsche, the filtration residue was further washed 4 times with 70 L of pure water, neutralized with aqueous ammonia, and placed in a polybucket having a volume of 45 L, pure water was added thereto, and the mixture was stirred (stirring speed: 300 rpm) with a three-one motor (type BL1200, manufactured by HEIDON, three turbine blade, blade diameter: about 8 cm), thereby obtaining a cellulose dispersion liquid having a concentration of 18%. This cellulose dispersion liquid was spray-dried (dispersion liquid supply rate: 31 kg/hour, inlet temperature: 180°C or higher and 220°C or lower, outlet temperature: 90°C or higher and 110°C or lower), thereby obtaining cellulose powder L.

[Comparative Example 6]

(Production of cellulose powder M)

3 kg of commercially available pulp (average degree of polymerization: 1,296) that had been shredded and 30 L of a 0.045 mol/L hydrochloric acid aqueous solution were placed in a low-speed type stirrer (manufactured by NGK Chemitech., Ltd, 30 LGL reactor) and hydrolyzed at 115°C for 75 minutes while being stirred at a stirring speed of 210 rpm, thereby obtaining an acid-insoluble residue. The obtained acid-insoluble residue was filtered using a Nutsche so that the solid content concentration reached 33% by mass, thereby obtaining a filtration residue. Thereafter, the mixture was put into a stainless steel tank having a volume of 20 L, pure water was added thereto, and the mixture was stirred with a stirrer (HANWA AGITATOR, manufactured by Hanwa Engineering Co., Ltd., stirring blade diameter: about 10 cm) at a stirring speed of 450 rpm to obtain a cellulose dispersion liquid having a solid content concentration of 9.8% by mass. Further, the cellulose dispersion liquid was further neutralized with aqueous ammonia. The average particle diameter of the cellulose particles in the cellulose dispersion liquid was 26 μm. This cellulose dispersion liquid was spray-dried (dispersion liquid supply rate: 20 kg/hour, inlet temperature: 180°C or higher and 220°C or lower, outlet temperature: 90°C or higher and 110°C or lower), thereby obtaining cellulose powder M.

[Comparative Example 7]

(Production of cellulose powder N)

The cellulose powder H obtained in Comparative Example 1 was pulverized with an ultracentrifugal pulverizer, thereby obtaining cellulose powder N.

[Comparative Example 8]

(Production of cellulose powder O)

2 kg of commercially available pulp (average degree of polymerization: 1,030) that had been shredded was immersed in water and allowed to pass through a cutter mill (microcut head/blade gap: 2.029 mm, impeller rotation speed: 9,000 rpm) in a state of containing about 70% of moisture, pure water was added thereto to prepare a cellulose dispersion liquid having a concentration of about 2%, and the dispersion liquid was treated 6 times with a high-pressure homogenizer (treatment pressure: 200 MPa) and centrifuged at a centrifugal force of 19,600 m/s2 to discard the supernatant, thereby obtaining a precipitate. About 2 kg of the precipitate dried at 40°C for 16 hours and 30 L of a 4N hydrochloric acid aqueous solution were placed in a low-speed type stirrer (manufactured by NGK Chemitech., Ltd, 50 LGL reactor), and hydrolyzed at 40°C for 48 hours while being stirred, thereby obtaining an acid-insoluble residue. The obtained acid-insoluble residue was filtered using a Nutsche, thereby obtaining a filtration residue. Thereafter, the filtration residue was placed in a polybucket having a volume of 90 L, pure water was added thereto, and the mixture was stirred with a 3-1 motor, thereby obtaining a cellulose dispersion liquid having a solid content concentration of 15% by mass. Further, the cellulose dispersion liquid was further neutralized with aqueous ammonia. The average particle diameter of the cellulose particles in the cellulose dispersion liquid was 18 $\mu$m. This cellulose dispersion liquid was spray-dried (dispersion liquid supply rate: 6 kg/hour, inlet temperature: 180°C or higher and 220°C or lower, outlet temperature: 50°C or higher and 70°C or lower), thereby obtaining cellulose powder O.

The evaluation results of the powder physical properties of each cellulose powder are listed in Tables 3 and 4.

[Table 3]

|  | Ex. 1 | Ex. 2 | Ex. 3 | Ex. 4 | Ex. 5 | Ex. 6 | Ex. 7 |
|---|---|---|---|---|---|---|---|
| Cellulose powder | A | B | C | D | E | F | G |
| Average degree of polymerization [-] | 249 | 231 | 292 | 269 | 253 | 250 | 320 |
| Average **particle** diameter $D_{50}$ [$\mu$m] | 38 | 46 | 60 | 42 | 37 | 36 | 50 |
| Loose bulk density X [g/cm$^3$] | 0.08 | 0.11 | 0.1 | 0.09 | 0.11 | 0.11 | 0.13 |
| Water absorption rate Cv [g$^2$/s] | 8.7 | 8.2 | 8.1 | 9.7 | 6.2 | 8.2 | 6.5 |
| Cv/$D_{50}$ [g$^2$/s·$\mu$m] | 0.23 | 0.18 | 0.13 | 0.23 | 0.17 | 0.23 | 0.13 |
| 1.5X - 0.06 | 0.07 | 0.11 | 0.09 | 0.07 | 0.17 | 0.23 | 0.13 |
| Cohesive force F [N%] | 58 | 56 | 63 | 69 | 63 | 71 | 62 |
| F/$D_{50}$ [N%/$\mu$m] | 1.52 | 1.23 | 1.04 | 1.66 | 1.71 | 1.95 | 1.24 |
| Powder dynamic friction angle [°] | 51 | 49 | 50 | 51 | 54 | 53 | 52 |
| Compression ratio [%] | 63 | 55 | 58 | 60 | 57 | 58 | 60 |
| Primary particle ratio [%] | 30 | 27 | 28 | 25 | 36 | 40 | 26 |
| L/D of primary particles [-] | 4.2 | 4.0 | 4.0 | 4.3 | 3.9 | 4.0 | 3.8 |

[Table 4]

|  | Com. Ex. 1 | Com. Ex. 2 | Com. Ex. 3 | Com. Ex. 4 | Com. Ex. 5 | Com. Ex. 6 | Com. Ex. 7 | Com. Ex. 8 |
|---|---|---|---|---|---|---|---|---|
| Cellulose powder | H | I | J | K | L | M | N | O |
| Average degree of polymerization [-] | 300 | 276 | 225 | 192 | 185 | 270 | 205 | 210 |
| Average particle diameter $D_{50}$ [$\mu$m] | 47 | 58 | 32 | 45 | 95 | 53 | 16 | 127 |
| Loose bulk density X [g/cm$^3$] | 0.13 | 0.18 | 0.18 | 0.27 | 0.31 | 0.18 | 0.17 | 0.26 |
| Water absorption rate Cv [g$^2$/s] | 5.3 | 5.3 | 3.6 | 3.6 | 4.5 | 7.2 | 2.0 | 2.9 |
| Cv/$D_{50}$ [g$^2$/s·$\mu$m] | 0.11 | 0.09 | 0.11 | 0.08 | 0.05 | 0.14 | 0.13 | 0.02 |
| 1.5X - 0.06 | 0.13 | 0.21 | 0.20 | 0.34 | 0.40 | 0.21 | 0.20 | 0.33 |
| Cohesive force F [N%] | 65 | 40 | 41 | 9 | 26 | 44 | 31 | 27 |
| F/$D_{50}$ [N%/$\mu$m] | 1.38 | 0.68 | 1.28 | 0.19 | 0.27 | 0.82 | 2.00 | 0.21 |
| Powder dynamic friction angle [°] | 50 | 49 | 49 | 42 | 38 | 49 | 49 | 47 |
| Compression ratio [%] | 59 | 49 | 53 | 40 | 30 | 47 | 58 | 33 |
| Primary particle ratio [%] | 27 | 11 | 11 | 3 | 3 | 15 | 50 | 19 |
| L/D of primary particles [-] | 3.7 | 3.5 | 3.7 | 3.3 | 3.6 | 3.6 | 2.7 | 3.5 |

The evaluation results in a case where each cellulose powder was formed into a tablet are listed in Tables 5 to 7.

Further, according to the standards for determining the tablet obtained from each cellulose powder listed in the tables, a tablet having an abrasion degree of less than 0.5% by mass is evaluated as A, a tablet having an abrasion degree of 0.5% by mass or greater and less than 1.0% by mass is evaluated as B, and a tablet having an abrasion degree of 1.0% by mass or greater is evaluated as C.

[Table 5]

| 410 mg tablet | | | | | | | |
|---|---|---|---|---|---|---|---|
| | Cellulose powder | Pressure [kN] | Mass CV [mass%] | Hardness [N] | Disintegration time [sec] | Abrasion degree | |
| | | | | | | [mass%] | Determination |
| Ex. 1 | A | - | - | - | - | - | - |
| Ex. 2 | B | 13 | 1.0 | 56 | 264 | 0.4 | A |
| Ex. 3 | C | 17 | 0.6 | 79 | 1041 | 0.4 | A |
| Ex. 4 | D | 14 | 0.9 | 63 | 980 | 0.4 | A |
| Ex. 5 | E | 17 | 0.6 | 75 | 449 | 0.3 | A |
| Com. Ex. 1 | H | - | - | - | - | - | - |
| Com. Ex. 2 | I | 13 | 0.4 | 40 | 43 | 0.6 | B |
| Com. Ex. 3 | J | 14 | 1.0 | 51 | 119 | 0.6 | B |
| Com. Ex. 4 | K | 12 | 0.5 | 32 | 21 | 0.8 | B |
| Com. Ex. 5 | L | 17 | 0.5 | 21 | 18 | 19.4 | C |

[Table 6]

| 370 mg tablet | | | | | | | |
|---|---|---|---|---|---|---|---|
| | Cellulose powder | Pressure [kN] | Mass CV [mass%] | Hardness [N] | Disintegration time [sec] | Abrasion degree | |
| | | | | | | [mass%] | Determination |
| Ex. 1 | A | 14 | 0.8 | 51 | 1457 | 0.4 | A |
| Ex. 2 | B | 17 | 0.9 | 44 | 408 | 0.4 | A |
| Ex. 3 | C | 18 | 0.9 | 50 | 1058 | 0.4 | A |
| Ex. 4 | D | 20 | 0.6 | 55 | 1425 | 0.1 | A |
| Ex. 5 | E | 16 | 0.5 | 53 | 308 | 0.4 | A |
| Example 7 | G | 17 | 0.8 | 53 | 1755 | 0.4 | A |
| Com. Ex. 1 | H | 17 | 1.3 | 50 | 566 | 0.6 | B |
| Com. Ex. 2 | I | 24 | 0.7 | 38 | 178 | 5.3 | C |
| Com. Ex. 3 | J | 17 | 0.5 | 33 | 112 | 6.1 | C |
| Com. Ex. 4 | K | 16 | 0.5 | 15 | 19 | 17.8 | C |
| Com. Ex. 5 | L | 15 | 0.5 | 11 | 17 | 23.3 | C |

[Table 7]

| 140 mg tablet | | | | | | | |
|---|---|---|---|---|---|---|---|
| | Cellulose powder | Pressure [kN] | Mass CV [mass%] | Hardness [N] | Disintegration time [sec] | Abrasion degree | |
| | | | | | | [mass%] | Determination |
| Ex. 4 | D | 5 | 0.3 | 118 | 1710 | 0.3 | A |
| Ex. 6 | F | 4 | 0.4 | 134 | 682 | 0.3 | A |
| Com. Ex. 5 | L | 6 | 0.1 | 114 | 69 | 0.6 | B |
| Com. Ex. 6 | M | 4 | 0.1 | 108 | 139 | 0.6 | B |
| Com. Ex. 7 | N | 3 | 0.4 | 101 | 21 | 0.7 | B |

(continued)

| 140 mg tablet | | | | | | | |
|---|---|---|---|---|---|---|---|
| | Cellulose powder | Pressure [kN] | Mass CV [mass%] | Hardness [N] | Disintegration time [sec] | Abrasion degree | |
| | | | | | | [mass%] | Determination |
| Com. Ex. 8 | O | 6 | 0.2 | 121 | 75 | 0.5 | B |

In Tables 5 to 7, in the tablets formed of the cellulose powders B to E (Examples 2 to 5), which were "cellulose powder in which the ratio Y (Cv/$D_{50}$) of the water absorption rate Cv to the average particle diameter $D_{50}$ is 0.12 g$^2$/(s·μm) or greater and the loose bulk density X was 0.135 g/cm$^3$ or less" or "cellulose powder in which the relationship between the ratio Y (Cv/$D_{50}$) of the water absorption rate Cv to the average particle diameter $D_{50}$ and the loose bulk density X satisfied Y ≥ 1.5 × X - 0.06 (Expression (1-1))", the variation in tablet mass was small and the hardness and the abrasion were excellent while the disintegrability was satisfactorily maintained in 410 mg tablets. Even in 370 mg tablets formed of the cellulose powders A to E (Examples 1 to 5), the variation in tablet mass was small, and the hardness and the abrasion were excellent while the disintegrability was satisfactorily maintained. Even in 470 mg tablets formed of the cellulose powders D and F (Examples 4 and 6), the variation in tablet mass was small, and the hardness and the abrasion were excellent while the disintegrability was satisfactorily maintained. In this manner, in a case of producing tablets using the cellulose powders A to F (Examples 1 to 6), it was possible to obtain tablets having excellent hardness and abrasion degree while properties of a small variation in tablet mass and satisfactory disintegrability were maintained and it was possible to further reduce the size of the tablets without changing the amount of the active component such as a drug. In a case of producing tablets in the same manner as described above using the cellulose powder G (Example 7), tablets having a small variation in tablet mass, excellent hardness and abrasion, and satisfactory disintegrability were obtained in the same manner as in a case where the cellulose powders A to F (Examples 1 to 6) were used.

In addition, based on comparison between the cellulose powders A and D (Examples 1 and 4) having the same average particle diameter $D_{50}$, the same average degree of polymerization, and the same ratio Cv/$D_{50}$ and different ratios F/$D_{50}$, the cellulose powder D (Example 4) in which F/$D_{50}$ was larger ($D_{50}$ was the same, that is, the value of the cohesive force F was larger) tended to have a smaller abrasion degree.

Further, based on comparison between the cellulose powders D and F (Examples 4 and 6) having the same average particle diameter $D_{50}$, the same average degree of polymerization, the same ratio Cv/$D_{50}$, different powder dynamic friction angles, and different primary particle ratios, the cellulose powder F (Example 6) having a larger powder dynamic friction angle and a larger primary particle ratio tended to have more satisfactory disintegrability.

Meanwhile, in the cellulose powders H to L (Comparative Examples 1 to 5) in which "the ratio Y (Cv/$D_{50}$) of the water absorption rate Cv to the average particle diameter $D_{50}$ was less than 0.12 g$^2$/(s·μm) or the loose bulk density X was greater than 0.135 g/cm$^3$" and "the relationship between the ratio Y (Cv/$D_{50}$) of the water absorption rate Cv to the average particle diameter $D_{50}$ and the loose bulk density X did not satisfy Cv ≥ 1.5 × X - 0.06", the variation in the tablet mass was satisfactory in the 410 mg tablets, but the hardness was less than 40 N, which was poor, in the tablets formed of the cellulose powders K and L (Comparative Examples 4 and 5), and the hardness was not less than 0.5% by mass in the tablets formed of the cellulose powders I and J (Comparative Examples 2 and 3). In the 370 mg tablets formed of the cellulose powders I to L (Comparative Examples 2 to 5), the hardness was less than 40 N, which was poor. In the 370 mg tablet formed of the cellulose powder H (Comparative Example 1), the abrasion degree was 0.5% by mass or greater. In the 470 mg tablets formed of the cellulose powders L to O (Comparative Examples 5 to 8), the abrasion degree was not less than 0.5% by mass.

INDUSTRIAL APPLICABILITY

According to the cellulose powder of the present embodiment, it is possible to provide cellulose powder which is capable of obtaining a smaller molded article having excellent hardness and a low abrasion degree without changing the amount of an active component such as a drug while the variation in mass is reduced and the disintegrability is satisfactorily maintained.

**Claims**

1. A cellulose powder,

   wherein a ratio Y (Cv/$D_{50}$) of a water absorption rate Cv to an average particle diameter $D_{50}$ is 0.12 g$^2$/(s·μm) or greater, and

a loose bulk density X is 0.135 g/cm$^3$ or less.

2. A cellulose powder,
   wherein a relationship between a ratio Y (Cv/D$_{50}$) of a water absorption rate Cv to an average particle diameter D$_{50}$ and a loose bulk density X satisfies Expression (1-1),

$$Y \geq 1.5 \times X - 0.06 \cdots\cdots \text{Expression (1-1)}.$$

3. The cellulose powder according to Claim 2,

   wherein the ratio Y (Cv/D$_{50}$) of the water absorption rate Cv to the average particle diameter D$_{50}$ is 0.12 g$^2$/(s·μm) or greater, and
   the loose bulk density X is 0.135 g/cm$^3$ or less.

4. The cellulose powder according to Claim 1 or 2,
   wherein a ratio F/D$_{50}$ of a cohesive force F to the average particle diameter D$_{50}$ is 0.01N%/μm or greater.

5. The cellulose powder according to Claim 1 or 2,
   wherein a powder dynamic friction angle is 30° or greater.

6. The cellulose powder according to Claim 1 or 2,
   wherein a primary particle ratio is 20% or greater.

7. The cellulose powder according to Claim 1 or 2,
   wherein a ratio L/D of primary particles is 2.0 or greater.

8. The cellulose powder according to Claim 1 or 2,
   wherein a compression ratio is 40% or greater.

9. The cellulose powder according to Claim 1 or 2,
   wherein an average degree of polymerization is 100 or greater and 450 or less.

10. The cellulose powder according to Claim 1 or 2,
    wherein the average particle diameter D$_{50}$ is 15 μm or greater and 300 μm or less.

11. The cellulose powder according to Claim 1 or 2,
    wherein the water absorption rate Cv is 2.0 g$^2$/s or greater and 12.0 g$^2$/s or less.

12. The cellulose powder according to Claim 1 or 2,
    wherein a cohesive force F is 30 N% or greater and 100 N% or less.

13. A molded article comprising:

    one or more active components; and
    the cellulose powder according to Claim 1 or 2.

14. The molded article according to Claim 13,
    wherein the active component is a pharmaceutically active component.

15. The molded article according to Claim 13,
    wherein the active component is an active component for food.

16. The molded article according to Claim 13,
    wherein the molded article is a tablet.

## INTERNATIONAL SEARCH REPORT

| International application No. |
| --- |
| **PCT/JP2024/013018** |

**A.　CLASSIFICATION OF SUBJECT MATTER**

*C08J 3/12*(2006.01)i; *A23L 29/262*(2016.01)i; *A61K 9/20*(2006.01)i; *A61K 31/375*(2006.01)i; *A61K 47/38*(2006.01)i
FI:　C08J3/12 Z; A23L29/262; A61K9/20; A61K31/375; A61K47/38

According to International Patent Classification (IPC) or to both national classification and IPC

**B.　FIELDS SEARCHED**

Minimum documentation searched (classification system followed by classification symbols)

C08J3/12; A23L29/262; A61K9/20; A61K31/375; A61K47/38

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

**C.　DOCUMENTS CONSIDERED TO BE RELEVANT**

| Category* | Citation of document, with indication, where appropriate, of the relevant passages | Relevant to claim No. |
| --- | --- | --- |
| A | WO 2016/024493 A1 (ASAHI KASEI KABUSHIKI KAISHA) 18 February 2016 (2016-02-18)<br>claims, paragraphs [0022], [0035]-[0049], examples 1-9 | 1-16 |
| A | WO 2021/090421 A1 (ASAHI KASEI KABUSHIKI KAISHA) 14 May 2021 (2021-05-14)<br>claims, paragraphs [0027]-[0037], examples 1-12 | 1-16 |
| A | JP 2022-146628 A (ASAHI KASEI KABUSHIKI KAISHA) 05 October 2022 (2022-10-05)<br>claims, paragraphs [0043]-[0052], [0099]-[0105], table 1 | 1-16 |
| A | WO 2019/130701 A1 (ASAHI KASEI KABUSHIKI KAISHA) 04 July 2019 (2019-07-04)<br>claims, paragraphs [0022]-[0039], example 2 | 1-16 |

☐ Further documents are listed in the continuation of Box C.　　☑ See patent family annex.

| | |
| --- | --- |
| \* Special categories of cited documents:<br>"A" document defining the general state of the art which is not considered to be of particular relevance<br>"D" document cited by the applicant in the international application<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
| --- | --- |
| **13 June 2024** | **25 June 2024** |

| Name and mailing address of the ISA/JP | Authorized officer |
| --- | --- |
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (July 2022)

**INTERNATIONAL SEARCH REPORT**
Information on patent family members

International application No.

**PCT/JP2024/013018**

| Patent document cited in search report | | | Publication date (day/month/year) | Patent family member(s) | | | Publication date (day/month/year) |
|---|---|---|---|---|---|---|---|
| WO | 2016/024493 | A1 | 18 February 2016 | US | 2017/0258728 | A1 | |
| | | | | claims, paragraphs [0025], [0039]-[0055], examples 1-9 | | | |
| | | | | EP | 3181616 | A1 | |
| | | | | TW | 201613646 | A | |
| WO | 2021/090421 | A1 | 14 May 2021 | US | 2023/0059899 | A1 | |
| | | | | claims, paragraphs [0051]-[0063], examples 1-12 | | | |
| | | | | EP | 4056638 | A1 | |
| | | | | CN | 112770779 | A | |
| | | | | CA | 3158393 | A | |
| | | | | BR | 112022007434 | A | |
| JP | 2022-146628 | A | 05 October 2022 | (Family: none) | | | |
| WO | 2019/130701 | A1 | 04 July 2019 | TW | 201927340 | A | |

Form PCT/ISA/210 (patent family annex) (July 2022)

**REFERENCES CITED IN THE DESCRIPTION**

**Patent documents cited in the description**

- JP 2023058328 A
- JP 2020121298 A

- JP 6273052 B

**Non-patent literature cited in the description**

- Encyclopedia of Pharmaceutical Excipients. Yakuji Nippo, Ltd.